# EUROPEAN PATENT APPLICATION

(11) **EP 4 556 072 A2**
(43) Date of publication of application: **21.05.2025**
(21) Application number: 25167193.9
(22) Date of filing: 20.08.2020
(51) Int. Cl.: A61P 37/02

(54) **COMPOSITIONS FOR MONOCYTE AND MACROPHAGE POLARIZATION AND METHODS OF USE**

(30) Priority: 23.08.2019 US 201962891188 P
(62) Divisional of application: 20776222.0
(71) Applicant: Orbsen Therapeutics Limited, Galway H91 A3EF (IE)
(72) Inventor: ELLIMAN, Stephen. J., Galway, H91 A3EF (IE); ALAGESAN, Senthilkumar, Galway, H91 A3EF (IE); DEEDIGAN, Laura, M., Galway, H91 A3EF (IE)
(74) Representative: Schlich

(57) **Abstract**

Provided herein are methods and compositions for monocyte and macrophage polarization including methods for use in treating a disease or disorder.

## Description

### CROSS-REFERENCE TO RELATED APPLICATIONS

This application claims the benefit of U.S. Provisional Patent Application No. 62/891,188 filed August 23, 2019, which is incorporated herein by reference in its entirety.

### BACKGROUND

Mitochondrial function has been recognized to decline during aging concomitant with mitochondrial morphological changes such as abnormally rounded mitochondria and reduced mitochondrial numbers and decreases in mitochondrial DNA. Further, it is recognized that mitochondrial dysfunction plays a role in onset of various diseases. Restoration of mitochondrial numbers and function has the potential to treat these diseases and reverse some of the effects of aging on cellular function and metabolism.

### SUMMARY

Provided herein are methods of treating an inflammatory disease in an individual. The methods comprise: administering a population of stromal stem cells to the individual in an amount effective to reduce at least one symptom of the inflammatory disease, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47. In some embodiments, the population of mammalian stromal stem cells has reduced CDC50 activity. In some embodiments, the population of mammalian stromal stem cells has increased IRG1/ACOD1 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. Alternatively and/or additionally, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and the methods comprise administering to the individual a population of mammalian stromal stem cells, wherein the population of mammalian stromal stem cells has reduced CDC50 activity. In some embodiments, the population of mammalian stromal stem cells has reduced CDC50 activity. In some embodiments, the population of mammalian stromal stem cells has increased IRG1/ACOD1 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and the methods comprise administering to the individual a population of mammalian stromal stem cells, wherein the population of mammalian stromal stem cells has increased IRG1/ACOD1 activity. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and the methods comprise administering to the individual a population of mammalian stromal stem cells, wherein the population of mammalian stromal stem cells has increased expression of PGC1α. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and the methods comprise administering to the individual a population of mammalian stromal stem cells, wherein the population of mammalian stromal stem cells has reduced expression of CD47. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and methods comprises administering to the individual a population of mammalian stromal stem cells, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47; and wherein at least 30% of the population of mammalian stromal stem cells is positive for SDC2. It is preferred that the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of preparing a population of stromal stem cells, and the methods comprise a) obtaining a population of mammalian cells; b) treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47; and c) isolating SDC2+ cells from the treated population of mammalian cells. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. In some embodiments, the stromal stem cell is a human cell, a mouse cell, a rat cell, or an equine cell. In some embodiments, the population of mammalian cells is obtained from a source selected from bone marrow, umbilical cord, umbilical cord blood, adipose tissue, skeletal muscle, endometrium, placenta, Wharton's jelly, and cells derived from pluripotent cells. In some embodiments, the population of mammalian cells is exposed to a low oxygen environment. In some embodiments, isolating SDC2+ cells from the treated population of mammalian cells comprises contacting the treated population of mammalian cells to an SDC2 binding agent and isolating cells bound to the SDC2 binding agent. In such embodiments, the SDC2 binding agent may comprise an anti-SDC2 antibody or fragment thereof. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell.

Also provided herein are methods of treating an individual, and methods comprise administering to the individual a composition comprising exosomes isolated from a population of mammalian stromal stem cells, wherein the population of mammalian stromal stem cells has reduced expression of CDC50. In some embodiments, the population of mammalian stromal stem cells has increased IRG1/ACOD1 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and methods comprise administering to the individual a composition comprising exosomes isolated from a population of mammalian stromal stem cells, wherein the population of mammalian stromal stem cells has increased expression of IRG1/ACOD1. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and methods comprise administering to the individual a composition comprising exosomes isolated from a population of mammalian stromal stem cells, wherein the population of mammalian stromal stem cells has increased expression of PGC1α. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and methods comprise administering to the individual a composition comprising exosomes isolated from a population of mammalian stromal stem cells, wherein the population of mammalian stromal stem cells has reduced expression of CD47. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and methods comprise administering to the individual a composition comprising exosomes isolated from a population of mammalian stromal stem cells, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47; and wherein at least 30% of the population of mammalian stromal stem cells is positive for SDC2. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of preparing a composition comprising exosomes isolated from a population of stromal stem cells, and methods comprise a) obtaining a population of mammalian cells; b) treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47; and c) isolating SDC2+ exosomes from the treated population of mammalian cells. In some embodiments, the population of mammalian stromal stem cells has reduced CDC50 activity. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. In some embodiments, the stromal stem cell is a human cell, a mouse cell, a rat cell, or an equine cell. In some embodiments, the population of mammalian cells is obtained from a source selected from bone marrow, umbilical cord, umbilical cord blood, adipose tissue, skeletal muscle, endometrium, placenta, Wharton's jelly, and cells derived from pluripotent cells. In some embodiments, the population of mammalian cells is exposed to a low oxygen environment. In some embodiments, isolating SDC2+ cells from the treated population of mammalian cells comprises contacting the treated population of mammalian cells to an SDC2 binding agent and isolating cells bound to the SDC2 binding agent. In such embodiments, the SDC2 binding agent may comprise an anti-SDC2 antibody or fragment thereof. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell.

Also provided herein are methods of measuring a potency of a batch of stromal stem cells, the methods comprise(a) isolating a population of SDC2+ stromal stem cells from a mixed population of mammalian cells; and (b) measuring at least one of (i) an expression level of IRG1/ACOD1 and (ii) a release level of itaconate. In some embodiments, the expression level of IRG1/ACOD1 is measured by QPCR or quantitative FACS. In some embodiments, the release level of itaconate is measured by mass spectrometry. In some embodiments, the mixed population of mammalian cells is bone marrow cells or umbilical cord cells. In some embodiments, the mixed population of mammalian cells is human. In some embodiments, the method further comprises treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47. In such embodiments, it is contemplated that wherein the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. In some embodiments, the stromal stem cell is a human cell, a mouse cell, a rat cell, or an equine cell. In some embodiments, the population of mammalian cells is obtained from a source selected from bone marrow, umbilical cord, umbilical cord blood, adipose tissue, skeletal muscle, endometrium, placenta, Wharton's jelly, and cells derived from pluripotent cells. In some embodiments, the population of mammalian cells is exposed to a low oxygen environment. In some embodiments, isolating SDC2+ cells from the treated population of mammalian cells comprises contacting the treated population of mammalian cells to an SDC2 binding agent and isolating cells bound to the SDC2 binding agent. In such embodiments, the SDC2 binding agent may comprise an anti-SDC2 antibody or fragment thereof. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

An understanding of the features and advantages of the present invention will be obtained by reference to the following detailed description that sets forth illustrative embodiments, in which the principles of the invention are utilized, and the accompanying drawings of which:
**Figs. 1A-D**show correlation between the amount of indoleamine 2,3-dioxygenase (IDO) produced and the T cell inhibitory potential of MSC donors.
**Figs. 2A-D**show that IDO-1^{hi} DP donor is capable of inducing immune regulatory monocytes in comparison to IDO-1^{lo}DP donor in a 24hr monocyte assay.
**Figs. 3A-C**show that reduction in TMEM30A expression results in significantly increased PtS on the cell surface of ORBCEL-C.
**Figs. 4A-C**show impact of siTMEM30A on ORBCEL-C phenotype.
**Figs. 5A-B**show viability and morphology of siTMEM30A-treated ORBCEL-C.
**Figs. 6A-D**show that reduction in TMEM30A expression and increased PtS results in higher IDO-1 expression (converts IDO-1^{lo} to IDO-1^{hi}
**Figs. 7A-C**show that TMEM30A KD Switches IDO-1^{lo} donor's M2 monocytes induction from Low to High (CA49DP = IDO-1^{hi} & CA58DP = IDO-1^{lo}).
**Figs. 8A-C**show that induction of regulatory monocytes (CD163⁺ CD206⁺) by IDO-1^{hi} DP is via an active cell-cell interaction process.
**Figs. 9A-C**show that IDO-1^{hi} donor primed monocytes are capable of inducing regulatory T-cells (T-regs).

### DETAILED DESCRIPTION

Stromal stem cells have shown utility in methods of treatment for a variety of diseases spanning organ systems. In particular, stromal stem cells and exosomes isolated from stromal stem cells show promise in treating inflammatory disease such as inflammatory liver disease, inflammatory kidney disease, inflammatory lung disease, and skin ulcers. In particular, stromal stem cell populations having at least 30% SDC2+ cells and exosomes isolated from these cells are thought to be useful therapeutics for disease.

A mechanism of action for SDC2+ stromal stem cell and exosome compositions has long eluded researchers. Disclosed herein is the discovery that the therapeutic effect of stromal stem cells and exosomes isolated from stromal stem cells is in delivery of mitochondria to diseased cells and tissue. Further, it has been found that stromal stem cells improve monocyte and macrophage polarization to induce an M2 phenotype. Accordingly, SDC2+ stromal stem cells and exosomes therefrom treated to have increased capability of mitochondrial transfer have improved therapeutic efficacy over untreated SDC2+ stromal stem cells.

### Compositions for Monocyte and Macrophage Polarization

Provided herein are compositions comprising stromal stem cells and exosome compositions having increased capabilities of monocyte and macrophage polarization to M2 phenotype, such as SDC2+ stromal stem cells and SDC2+ exosomes treated to have at least one of increased mitochondrial numbers and increased mitochondrial density compared to untreated SDC2+ stromal stem cells and SDC2+ exosomes. Additionally, stromal stem cell and exosome compositions herein are treated to increase cell surface phosphatidylserine (PS). Further, stromal stem cell and exosome compositions herein are treated to have increased itaconate in the cells.

Compositions for increasing monocyte and macrophage polarization to M2 phenotype comprising stromal stem cells and exosomes, including SDC2+ stromal stem cells and SDC2+ exosomes, are treated to increase their capacity for monocyte and macrophage polarization to M2 phenotype. Non-limiting examples of modifying SDC2+ stromal stem cells and SDC2+ exosomes include but are not limited to decreasing expression of CDC50, inhibiting CDC50 activity, increasing expression of IRG1/ACOD1, increasing IRG1/ACOD1 activity, decreasing expression of CD47, inhibiting CD47, increasing expression of PGC1α, and increasing PGC1α activity, including combinations of the above modifications. In some cases, stromal stem cells are isolated from bone marrow or umbilical cord tissue. In some cases, compositions for mitochondrial transfer comprising SDC2+ stromal stem cells and SDC2+ exosomes are treated to decrease expression of CDC50 or treated to inhibit CDC50 activity. Alternatively or in combination, compositions for mitochondrial transfer comprising SDC2+ stromal stem cells and SDC2+ exosomes are treated to increase expression of IRG1/ACOD1 or treated to increase IRG1/ACOD1 activity. Alternatively or in combination, compositions for mitochondrial transfer comprising SDC2+ stromal stem cells and SDC2+ exosomes are treated to decrease expression of CD47 or treated to inhibit CD47 activity. Alternatively or in combination, compositions for mitochondrial transfer comprising SDC2+ stromal stem cells and SDC2+ exosomes are treated to increase expression of PGC1α or treated to increase PGC1α activity. Exemplary treatments for decreasing expression of CDC50 and/or CD47 include but are not limited to gene knockout and RNAi methods, using CRISPR or siRNA techniques disclosed elsewhere herein. Exemplary treatments for increasing expression of PGC1α and/or IRG1/ACOD1 include but are not limited to transfecting or transducing the cells with a plasmid or a virus that overexpresses PGC1α and/or IRG1/ACOD1.

In certain aspects, provided herein are compositions for increasing monocyte and macrophage polarization to M2 phenotype comprising a stromal stem cell treated to decrease expression of or inhibit CDC50. In some cases, the stromal stem cell is an SDC2+ stromal stem cell. In some cases, the stromal stem cell is treated with a CDC50 siRNA. In some cases, the stromal stem cell is treated with a virus expressing a shRNA targeting CDC50. In some cases, the stromal stem cell is treated with a CDC50 inhibitor. In some cases, CDC50 expression is reduced by 10% in a treated stromal stem cell compared to an untreated stromal stem cell. In some cases, CDC50 expression or activity is reduced by 20% in a treated stromal stem cell compared to an untreated stromal stem cell. In some cases, CDC50 expression or activity is reduced by 30% in a treated stromal stem cell compared to an untreated stromal stem cell. In some cases, CDC50 expression or activity is reduced by 40% in a treated stromal stem cell compared to an untreated stromal stem cell. In some cases, CDC50 expression or activity is reduced by 50% in a treated stromal stem cell compared to an untreated stromal stem cell. In some cases, CDC50 expression or activity is reduced by 60% in a treated stromal stem cell compared to an untreated stromal stem cell. In some cases, CDC50 expression or activity is reduced by 70% in a treated stromal stem cell compared to an untreated stromal stem cell. In some cases, CDC50 expression or activity is reduced by 80% in a treated stromal stem cell compared to an untreated stromal stem cell. In some cases, CDC50 expression or activity is reduced by 90% in a treated stromal stem cell compared to an untreated stromal stem cell. In some cases, CDC50 expression or activity is reduced by 95% in a treated stromal stem cell compared to an untreated stromal stem cell. In some cases, CDC50 expression or activity is reduced by 99% in a treated stromal stem cell compared to an untreated stromal stem cell. In some cases, CDC50 expression or activity is reduced by 100% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising a stromal stem cell treated to increase expression of or activate IRG1/ACOD1. In some cases, the stromal stem cell is an SDC2+ stromal stem cell. In some cases, the stromal stem cell is treated with an IRG1/ACOD1 plasmid. In some cases, the stromal stem cell is treated with a virus expressing IRG1/ACOD1. In some cases, the stromal stem cell is treated with an IRG1/ACOD1 agonist. In some cases, IRG1/ACOD1 expression is increased by 20% in a treated stromal stem cell compared to an untreated stromal stem cell. In some cases, IRG1/ACOD1 expression or activity is increased by 50% in a treated stromal stem cell compared to an untreated stromal stem cell. In some cases, IRG1/ACOD1 expression or activity is increased by 80% in a treated stromal stem cell compared to an untreated stromal stem cell. In some cases, IRG1/ACOD1 expression or activity is increased by 100% in a treated stromal stem cell compared to an untreated stromal stem cell. In some cases, IRG1/ACOD1 expression or activity is increased by 150% in a treated stromal stem cell compared to an untreated stromal stem cell. In some cases, IRG1/ACOD1 expression or activity is increased by 200% in a treated stromal stem cell compared to an untreated stromal stem cell. In some cases, IRG1/ACOD1 expression or activity is increased by 250% in a treated stromal stem cell compared to an untreated stromal stem cell. In some cases, IRG1/ACOD1 expression or activity is increased by 300% in a treated stromal stem cell compared to an untreated stromal stem cell. In some cases, IRG1/ACOD1 expression or activity is increased by 350% in a treated stromal stem cell compared to an untreated stromal stem cell. In some cases, IRG1/ACOD1 expression or activity is increased by 400% in a treated stromal stem cell compared to an untreated stromal stem cell. In some cases, IRG1/ACOD1 expression or activity is increased by 450% in a treated stromal stem cell compared to an untreated stromal stem cell. In some cases, IRG1/ACOD1 expression or activity is increased by 500% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising a stromal stem cell treated to decrease expression of or inhibit CD47. In some cases, the stromal stem cell is an SDC2+ stromal stem cell. In some cases, the stromal stem cell is treated with a CD47 siRNA. In some cases, the stromal stem cell is treated with a virus expressing an shRNA targeting CD47. In some cases, the stromal stem cell is treated with a CD47 inhibitor. In some cases, CD47 expression is reduced by 10% in a treated stromal stem cell compared to an untreated stromal stem cell. In some cases, CD47 expression or activity is reduced by 20% in a treated stromal stem cell compared to an untreated stromal stem cell. In some cases, CD47 expression or activity is reduced by 30% in a treated stromal stem cell compared to an untreated stromal stem cell. In some cases, CD47 expression or activity is reduced by 40% in a treated stromal stem cell compared to an untreated stromal stem cell. In some cases, CD47 expression or activity is reduced by 50% in a treated stromal stem cell compared to an untreated stromal stem cell. In some cases, CD47 expression or activity is reduced by 60% in a treated stromal stem cell compared to an untreated stromal stem cell. In some cases, CD47 expression or activity is reduced by 70% in a treated stromal stem cell compared to an untreated stromal stem cell. In some cases, CD47 expression or activity is reduced by 80% in a treated stromal stem cell compared to an untreated stromal stem cell. In some cases, CD47 expression or activity is reduced by 90% in a treated stromal stem cell compared to an untreated stromal stem cell. In some cases, CD47 expression or activity is reduced by 95% in a treated stromal stem cell compared to an untreated stromal stem cell. In some cases, CD47 expression or activity is reduced by 99% in a treated stromal stem cell compared to an untreated stromal stem cell. In some cases, CD47 expression or activity is reduced by 100% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising a stromal stem cell treated to increase expression of or activate PGC1α. In some cases, the stromal stem cell is an SDC2+ stromal stem cell. In some cases, the stromal stem cell is treated with a PGC1α plasmid. In some cases, the stromal stem cell is treated with a virus expressing PGC1α. In some cases, the stromal stem cell is treated with a PGC1α agonist. In some cases, PGC1α expression is increased by 20% in a treated stromal stem cell compared to an untreated stromal stem cell. In some cases, PGC1α expression or activity is increased by 50% in a treated stromal stem cell compared to an untreated stromal stem cell. In some cases, PGC1α expression or activity is increased by 80% in a treated stromal stem cell compared to an untreated stromal stem cell. In some cases, PGC1α expression or activity is increased by 100% in a treated stromal stem cell compared to an untreated stromal stem cell. In some cases, PGC1α expression or activity is increased by 150% in a treated stromal stem cell compared to an untreated stromal stem cell. In some cases, PGC1α expression or activity is increased by 200% in a treated stromal stem cell compared to an untreated stromal stem cell. In some cases, PGC1α expression or activity is increased by 250% in a treated stromal stem cell compared to an untreated stromal stem cell. In some cases, PGC1α expression or activity is increased by 300% in a treated stromal stem cell compared to an untreated stromal stem cell. In some cases, PGC1α expression or activity is increased by 350% in a treated stromal stem cell compared to an untreated stromal stem cell. In some cases, PGC1α expression or activity is increased by 400% in a treated stromal stem cell compared to an untreated stromal stem cell. In some cases, PGC1α expression or activity is increased by 450% in a treated stromal stem cell compared to an untreated stromal stem cell. In some cases, PGC1α expression or activity is increased by 500% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising an exosome treated to decrease expression of or inhibit CDC50. In some cases, the exosome is an SDC2+ exosome. In some cases, the exosome is isolated from a stromal stem cell is treated with a CDC50 siRNA. In some cases, the exosome is isolated from a stromal stem cell is treated with a virus expressing an shRNA targeting CDC50. In some cases, the exosome is isolated from a stromal stem cell is treated with a CDC50 inhibitor. In some cases, CDC50 expression is reduced by 10% in a treated stromal stem cell compared to an untreated stromal stem cell. In some cases, CDC50 expression or activity is reduced by 20% in a treated stromal stem cell compared to an untreated stromal stem cell. In some cases, CDC50 expression or activity is reduced by 30% in a treated stromal stem cell compared to an untreated stromal stem cell. In some cases, CDC50 expression or activity is reduced by 40% in a treated stromal stem cell compared to an untreated stromal stem cell. In some cases, CDC50 expression or activity is reduced by 50% in a treated stromal stem cell compared to an untreated stromal stem cell. In some cases, CDC50 expression or activity is reduced by 60% in a treated stromal stem cell compared to an untreated stromal stem cell. In some cases, CDC50 expression or activity is reduced by 70% in a treated stromal stem cell compared to an untreated stromal stem cell. In some cases, CDC50 expression or activity is reduced by 80% in a treated stromal stem cell compared to an untreated stromal stem cell. In some cases, CDC50 expression or activity is reduced by 90% in a treated stromal stem cell compared to an untreated stromal stem cell. In some cases, CDC50 expression or activity is reduced by 95% in a treated stromal stem cell compared to an untreated stromal stem cell. In some cases, CDC50 expression or activity is reduced by 99% in a treated stromal stem cell compared to an untreated stromal stem cell. In some cases, CDC50 expression or activity is reduced by 100% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising an exosome treated to increase expression of or activate IRG1/ACOD1. In some cases, the exosome is an SDC2+ exosome. In some cases, the exosome is isolated from a stromal stem cell is treated with an IRG1/ACOD1 plasmid. In some cases, the exosome is isolated from a stromal stem cell is treated with a virus expressing IRG1/ACOD1. In some cases, the exosome is isolated from a stromal stem cell is treated with an IRG1/ACOD1 agonist. In some cases, IRG1/ACOD1 expression is increased by 20% in a treated stromal stem cell compared to an untreated stromal stem cell. In some cases, IRG1/ACOD1 expression or activity is increased by 50% in a treated stromal stem cell compared to an untreated stromal stem cell. In some cases, IRG1/ACOD1 expression or activity is increased by 80% in a treated stromal stem cell compared to an untreated stromal stem cell. In some cases, IRG1/ACOD1 expression or activity is increased by 100% in a treated stromal stem cell compared to an untreated stromal stem cell. In some cases, IRG1/ACOD1 expression or activity is increased by 150% in a treated stromal stem cell compared to an untreated stromal stem cell. In some cases, IRG1/ACOD1 expression or activity is increased by 200% in a treated stromal stem cell compared to an untreated stromal stem cell. In some cases, IRG1/ACOD1 expression or activity is increased by 250% in a treated stromal stem cell compared to an untreated stromal stem cell. In some cases, IRG1/ACOD1 expression or activity is increased by 300% in a treated stromal stem cell compared to an untreated stromal stem cell. In some cases, IRG1/ACOD1 expression or activity is increased by 350% in a treated stromal stem cell compared to an untreated stromal stem cell. In some cases, IRG1/ACOD1 expression or activity is increased by 400% in a treated stromal stem cell compared to an untreated stromal stem cell. In some cases, IRG1/ACOD1 expression or activity is increased by 450% in a treated stromal stem cell compared to an untreated stromal stem cell. In some cases, IRG1/ACOD1 expression or activity is increased by 500% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising an exosome treated to decrease expression of or inhibit CD47. In some cases, the exosome is an SDC2+ exosome. In some cases, the exosome is isolated from a stromal stem cell is treated with a CD47 siRNA. In some cases, the exosome is isolated from a stromal stem cell is treated with a virus expressing an shRNA targeting CD47. In some cases, the exosome is isolated from a stromal stem cell is treated with a CD47 inhibitor. In some cases, CD47 expression is reduced by 10% in a treated stromal stem cell compared to an untreated stromal stem cell. In some cases, CD47 expression or activity is reduced by 20% in a treated stromal stem cell compared to an untreated stromal stem cell. In some cases, CD47 expression or activity is reduced by 30% in a treated stromal stem cell compared to an untreated stromal stem cell. In some cases, CD47 expression or activity is reduced by 40% in a treated stromal stem cell compared to an untreated stromal stem cell. In some cases, CD47 expression or activity is reduced by 50% in a treated stromal stem cell compared to an untreated stromal stem cell. In some cases, CD47 expression or activity is reduced by 60% in a treated stromal stem cell compared to an untreated stromal stem cell. In some cases, CD47 expression or activity is reduced by 70% in a treated stromal stem cell compared to an untreated stromal stem cell. In some cases, CD47 expression or activity is reduced by 80% in a treated stromal stem cell compared to an untreated stromal stem cell. In some cases, CD47 expression or activity is reduced by 90% in a treated stromal stem cell compared to an untreated stromal stem cell. In some cases, CD47 expression or activity is reduced by 95% in a treated stromal stem cell compared to an untreated stromal stem cell. In some cases, CD47 expression or activity is reduced by 99% in a treated stromal stem cell compared to an untreated stromal stem cell. In some cases, CD47 expression or activity is reduced by 100% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising an exosome treated to increase expression of or activate PGC1α. In some cases, the exosome is an SDC2+ exosome. In some cases, the exosome is isolated from a stromal stem cell is treated with a PGC1α plasmid. In some cases, the exosome is isolated from a stromal stem cell is treated with a virus expressing PGC1α. In some cases, the exosome is isolated from a stromal stem cell is treated with a PGC1α agonist. In some cases, PGC1α expression is increased by 20% in a treated stromal stem cell compared to an untreated stromal stem cell. In some cases, PGC1α expression or activity is increased by 50% in a treated stromal stem cell compared to an untreated stromal stem cell. In some cases, PGC1α expression or activity is increased by 80% in a treated stromal stem cell compared to an untreated stromal stem cell. In some cases, PGC1α expression or activity is increased by 100% in a treated stromal stem cell compared to an untreated stromal stem cell. In some cases, PGC1α expression or activity is increased by 150% in a treated stromal stem cell compared to an untreated stromal stem cell. In some cases, PGC1α expression or activity is increased by 200% in a treated stromal stem cell compared to an untreated stromal stem cell. In some cases, PGC1α expression or activity is increased by 250% in a treated stromal stem cell compared to an untreated stromal stem cell. In some cases, PGC1α expression or activity is increased by 300% in a treated stromal stem cell compared to an untreated stromal stem cell. In some cases, PGC1α expression or activity is increased by 350% in a treated stromal stem cell compared to an untreated stromal stem cell. In some cases, PGC1α expression or activity is increased by 400% in a treated stromal stem cell compared to an untreated stromal stem cell. In some cases, PGC1α expression or activity is increased by 450% in a treated stromal stem cell compared to an untreated stromal stem cell. In some cases, PGC1α expression or activity is increased by 500% in a treated stromal stem cell compared to an untreated stromal stem cell. In some cases, stromal stem cells are isolated from bone marrow or umbilical cord tissue.

Compositions comprising stromal stem cells for mitochondrial transfer are disclosed herein. Some compositions comprise stromal stem cells modified to have reduced CDC50 and/or CD47 activity or expression and/or increased IRG1/ACOD1 and/or PGC1α activity or expression in a therapeutically effective amount. In some compositions, the amount of modified stromal stem cells ranges from 10^3-10^8 modified stromal stem cells, for example 10^3, 10^4, 10^5, 10^6, 10^7, or 10^8 modified stromal stem cells, or more modified stromal stem cells in the composition. Modified stromal stem cell compositions, in some cases, are concentrated to be diluted by the individual or the health care provider prior to administration. In some cases, modified stromal stem cell compositions are diluted and ready to be administered by the individual or health care provider. In some cases, modified stromal stem cell compositions are contained in single use vials, syringes, or IV bags. In some cases, multiple doses are present in a single container.

Stromal stem cell compositions for increasing monocyte and macrophage polarization to M2 phenotype disclosed herein, in some cases, comprise a cryoprotectant or cryopreservative. Cryoprotectants include DMSO, glycerol, polyethylene glycol, propylene glycol, glycerine, polyvinylpyrolidone, sorbitol, dextran, trehalose, and commercial formulations such as CryoStor from Biolife solutions. Stromal stem cell compositions herein retain potency after being frozen using special freezing protocols. Special freezing protocols include flash freezing, programmable rate freezer, and freezing in an insulated container. The stromal stem cell compositions are in some cases frozen in buffer or culture media having an added cryoprotectant. Buffers include physiologically acceptable buffers such as phosphate buffer, histidine buffer, citrate buffer, acetate buffer, Hypothermasol from Biolife Solutions and other suitable.

Stromal stem cell compositions for increasing monocyte and macrophage polarization to M2 phenotype disclosed herein are formulated in a physiologically acceptable buffer and in some cases supplemented by at least one excipient. Non-limiting examples of excipients include sucrose, trehalose, polyethylene glycol, a polysaccharide, a carrier protein, an inert protein, dextran, hydroxyl ethyl starch (HETA), PEG-4000, gelatin, PLGA, Eudragit RS 100 Nanoparticles, and combinations thereof. Such stromal stem cell compositions are stored at a temperature determined to be most stable (i.e., wherein the stromal stem cell composition retains highest potency, or retains potency for the longest period of time, or otherwise optimizes a desired trait). In some cases, addition of at least one excipient allows the composition to retain potency, such as paracrine signaling potency, when stored at a higher temperature than otherwise would be possible.

While exosome compositions for increasing monocyte and macrophage polarization to M2 phenotype described herein, in some cases, are derived from cells, the exosome compositions do not necessarily comprise living cells. Cell-free exosome compositions, therefore, are non-tumorigenic, that is, they do not increase the susceptibility of a subject to developing a tumor or cancer, because they do not comprise cells capable of differentiating into tumor cells. In alternative compositions, the exosomes are supplemented with cells, such as mesenchymal stromal cells, that contribute to antiinflammatory activity or paracrine signaling activity of the compositions.

Compositions comprising a wide range of exosomes for increasing monocyte and macrophage polarization to M2 phenotype are disclosed herein. Some compositions comprise exosomes in a therapeutically effective amount. In some compositions, the amount of exosomes ranges from 10⁶-10⁸ exosomes, for example 10⁶, 10⁷, 10⁸, or more exosomes in the composition. In some cases, a therapeutically effective amount of exosomes ranges from 1 µg to 700 mg of exosomes, for example 1 µg, 10 µg, 20 µg, 50 µg, 100 µg, 150 µg, 200 µg, 250 µg, 500 µg, 750 µg, 1 mg, 2 mg, 3 mg, 4 mg, 5 mg, 6 mg, 7 mg, 10 mg, 20 mg, 50 mg, 100 mg, 200 mg, 300 mg, 400 mg, 500 mg, 600 mg, 700 mg, or more exosomes in the composition. Exosome compositions, in some cases, are concentrated to be diluted by the individual prior to administration. In some cases, exosome compositions are diluted and ready to be administered by the individual. In some cases, exosome compositions are contained in single use vials or syringes. In some cases, multiple doses are present in a single container.

Exosome compositions for increasing monocyte and macrophage polarization to M2 phenotype disclosed herein retain potency or activity, such as paracrine signaling activity, after being frozen or cryopreserved, In some cases without the use of a cryoprotectant. Cryoprotectants include DMSO, glycerol, polyethylene glycol, propylene glycol, glycerine, polyvinylpyrolidone, sorbitol, dextran, trehalose, and commercial formulations such as CryoStor from Biolife solutions. The exosome compositions also retain potency after being frozen without using special freezing protocols. Special freezing protocols include flash freezing, programmable rate freezer, and freezing in an insulated container. A benefit of the durability of the exosome compositions is that they are more easily frozen and are frozen without cryoprotectants, resulting in compositions that are more durable, more easily and cheaply made, and less likely to suffer from batch variation resulting from loss of activity due to a defect in freezing protocol or composition. The exosome compositions are in some cases frozen in buffer or culture media. Buffers include physiologically acceptable buffers such as phosphate buffer, histidine buffer, citrate buffer, acetate buffer, Hypothermasol from Biolife Solutions and other suitable buffers. In some cases exosome compositions disclosed herein are lyophilized.

Exosome compositions for increasing monocyte and macrophage polarization to M2 phenotype disclosed herein are formulated in a physiologically acceptable buffer and in some cases supplemented by at least one excipient. Non-limiting examples of excipients include sucrose, trehalose, polyethylene glycol, a polysaccharide, a carrier protein, an inert protein, dextran, hydroxyl ethyl starch (HETA), PEG-4000, gelatin, PLGA, Eudragit RS 100 Nanoparticles, and combinations thereof. Such exosome compositions are stored at a temperature determined to be most stable (i.e., wherein the exosome composition retains highest potency, or retains potency for the longest period of time, or otherwise optimizes a desired trait). In some cases, addition of at least one excipient allows the composition to retain potency, such as paracrine signaling potency, when stored at a higher temperature than otherwise would be possible.

### Methods of Preparing Compositions for Monocyte/Macrophage Polarization

Provided herein are methods of preparing compositions comprising stromal stem cells and exosome compositions having increased capabilities of monocyte and macrophage polarization to M2 phenotype, such as SDC2+ stromal stem cells and SDC2+ exosomes treated to have at least one of increased mitochondrial numbers, increased mitochondrial density, increased cell surface phosphatidylserine, and increased itaconate compared to untreated SDC2+ stromal stem cells and SDC2+ exosomes.

Methods herein for preparing compositions for increasing monocyte and macrophage polarization to M2 phenotype comprising stromal stem cells and exosomes, including SDC2+ stromal stem cells and SDC2+ exosomes, comprise treating stromal stem cells to increase their capacity for mitochondrial transfer. Non-limiting examples of methods for preparing compositions for mitochondrial transfer comprising SDC2+ stromal stem cells and SDC2+ exosomes include but are not limited to methods of decreasing expression of CDC50, methods of inhibiting CDC50 activity, methods of increasing expression of IRG1/ACOD1, and methods of increasing IRG1/ACOD1 activity, methods of decreasing expression of CD47, methods of inhibiting CD47, methods of increasing expression of PGC1α, and methods of increasing PGC1α activity, including combinations of the above methods. In some cases, methods for preparing compositions for mitochondrial transfer comprising SDC2+ stromal stem cells and SDC2+ exosomes comprise treating to decrease expression of CDC50 or comprise treating to inhibit CDC50 activity. Alternatively or in combination, methods for preparing compositions for mitochondrial transfer comprising SDC2+ stromal stem cells and SDC2+ exosomes comprise treating to increase expression of IRG1/ACOD1 or comprise treating to increase IRG1/ACOD1 activity. Alternatively or in combination, methods for preparing compositions for mitochondrial transfer comprising SDC2+ stromal stem cells and SDC2+ exosomes comprise treating to decrease expression of CD47 or comprise treating to inhibit CD47 activity. Alternatively or in combination, methods for preparing compositions for mitochondrial transfer comprising SDC2+ stromal stem cells and SDC2+ exosomes comprise treating to increase expression of PGC1α or comprise treating to increase PGC1α activity. Exemplary methods for treating to decrease expression of CDC50 and/or CD47 include but are not limited to gene knockout and RNAi methods, using CRISPR or siRNA techniques suitable for methods herein. Exemplary methods for treating to increase expression of PGC1α include but are not limited to transfecting or transducing the cells with a plasmid or a virus that overexpresses PGC1α.

In certain aspects, provided herein are methods for preparing compositions for increasing monocyte and macrophage polarization to M2 phenotype comprising a stromal stem cell, the method comprising treating a stromal stem cell to decrease expression of or inhibit CDC50. In some cases, the stromal stem cell is an SDC2+ stromal stem cell. In some cases, the method comprises treating the stromal stem cell with a CDC50 siRNA. In some cases, the method comprises treating the stromal stem cell with a virus expressing an shRNA targeting CDC50. In some cases, the method comprises treating the stromal stem cell with a CDC50 inhibitor. In some cases, CDC50 expression is reduced by 10% in a treated stromal stem cell compared to an untreated stromal stem cell. In some cases, CDC50 expression or activity is reduced by 20% in a treated stromal stem cell compared to an untreated stromal stem cell. In some cases, CDC50 expression or activity is reduced by 30% in a treated stromal stem cell compared to an untreated stromal stem cell. In some cases, CDC50 expression or activity is reduced by 40% in a treated stromal stem cell compared to an untreated stromal stem cell. In some cases, CDC50 expression or activity is reduced by 50% in a treated stromal stem cell compared to an untreated stromal stem cell. In some cases, CDC50 expression or activity is reduced by 60% in a treated stromal stem cell compared to an untreated stromal stem cell. In some cases, CDC50 expression or activity is reduced by 70% in a treated stromal stem cell compared to an untreated stromal stem cell. In some cases, CDC50 expression or activity is reduced by 80% in a treated stromal stem cell compared to an untreated stromal stem cell. In some cases, CDC50 expression or activity is reduced by 90% in a treated stromal stem cell compared to an untreated stromal stem cell. In some cases, CDC50 expression or activity is reduced by 95% in a treated stromal stem cell compared to an untreated stromal stem cell. In some cases, CDC50 expression or activity is reduced by 99% in a treated stromal stem cell compared to an untreated stromal stem cell. In some cases, CDC50 expression or activity is reduced by 100% in a treated stromal stem cell compared to an untreated stromal stem cell.

In further aspects, provided herein are methods for preparing compositions for increasing monocyte and macrophage polarization to M2 phenotype comprising a stromal stem cell, the method comprising treating the stromal stem cell to increase expression of or activate IRG1/ACOD1. In some cases, the stromal stem cell is an SDC2+ stromal stem cell. In some cases, the method comprises treating the stromal stem cell with an IRG1/ACOD1 plasmid. In some cases, the method comprises treating the stromal stem cell with a virus expressing IRG1/ACOD1. In some cases, the method comprises treating the stromal stem cell with an IRG1/ACOD1 agonist. In some cases, IRG1/ACOD1 expression is increased by 20% in a treated stromal stem cell compared to an untreated stromal stem cell. In some cases, IRG1/ACOD1 expression or activity is increased by 50% in a treated stromal stem cell compared to an untreated stromal stem cell. In some cases, IRG1/ACOD1 expression or activity is increased by 80% in a treated stromal stem cell compared to an untreated stromal stem cell. In some cases, IRG1/ACOD1 expression or activity is increased by 100% in a treated stromal stem cell compared to an untreated stromal stem cell. In some cases, IRG1/ACOD1 expression or activity is increased by 150% in a treated stromal stem cell compared to an untreated stromal stem cell. In some cases, IRG1/ACOD1 expression or activity is increased by 200% in a treated stromal stem cell compared to an untreated stromal stem cell. In some cases, IRG1/ACOD1 expression or activity is increased by 250% in a treated stromal stem cell compared to an untreated stromal stem cell. In some cases, IRG1/ACOD1 expression or activity is increased by 300% in a treated stromal stem cell compared to an untreated stromal stem cell. In some cases, IRG1/ACOD1 expression or activity is increased by 350% in a treated stromal stem cell compared to an untreated stromal stem cell. In some cases, IRG1/ACOD1 expression or activity is increased by 400% in a treated stromal stem cell compared to an untreated stromal stem cell. In some cases, IRG1/ACOD1 expression or activity is increased by 450% in a treated stromal stem cell compared to an untreated stromal stem cell. In some cases, IRG1/ACOD1 expression or activity is increased by 500% in a treated stromal stem cell compared to an untreated stromal stem cell.

In additional aspects, provided herein are methods for preparing compositions for increasing monocyte and macrophage polarization to M2 phenotype comprising a stromal stem cell, the method comprising treating a stromal stem cell to decrease expression of or inhibit CD47. In some cases, the stromal stem cell is an SDC2+ stromal stem cell. In some cases, the method comprises treating the stromal stem cell with a CD47 siRNA. In some cases, the method comprises treating the stromal stem cell with a virus expressing an shRNA targeting CD47. In some cases, the method comprises treating the stromal stem cell is treated with a CD47 inhibitor. In some cases, CD47 expression is reduced by 10% in a treated stromal stem cell compared to an untreated stromal stem cell. In some cases, CD47 expression or activity is reduced by 20% in a treated stromal stem cell compared to an untreated stromal stem cell. In some cases, CD47 expression or activity is reduced by 30% in a treated stromal stem cell compared to an untreated stromal stem cell. In some cases, CD47 expression or activity is reduced by 40% in a treated stromal stem cell compared to an untreated stromal stem cell. In some cases, CD47 expression or activity is reduced by 50% in a treated stromal stem cell compared to an untreated stromal stem cell. In some cases, CD47 expression or activity is reduced by 60% in a treated stromal stem cell compared to an untreated stromal stem cell. In some cases, CD47 expression or activity is reduced by 70% in a treated stromal stem cell compared to an untreated stromal stem cell. In some cases, CD47 expression or activity is reduced by 80% in a treated stromal stem cell compared to an untreated stromal stem cell. In some cases, CD47 expression or activity is reduced by 90% in a treated stromal stem cell compared to an untreated stromal stem cell. In some cases, CD47 expression or activity is reduced by 95% in a treated stromal stem cell compared to an untreated stromal stem cell. In some cases, CD47 expression or activity is reduced by 99% in a treated stromal stem cell compared to an untreated stromal stem cell. In some cases, CD47 expression or activity is reduced by 100% in a treated stromal stem cell compared to an untreated stromal stem cell.

In further aspects, provided herein are methods for preparing compositions for increasing monocyte and macrophage polarization to M2 phenotype comprising a stromal stem cell, the method comprising treating the stromal stem cell to increase expression of or activate PGC1α. In some cases, the stromal stem cell is an SDC2+ stromal stem cell. In some cases, the method comprises treating the stromal stem cell with a PGC1α plasmid. In some cases, the method comprises treating the stromal stem cell with a virus expressing PGC1α. In some cases, the method comprises treating the stromal stem cell with a PGC1α agonist. In some cases, PGC1α expression is increased by 20% in a treated stromal stem cell compared to an untreated stromal stem cell. In some cases, PGC1α expression or activity is increased by 50% in a treated stromal stem cell compared to an untreated stromal stem cell. In some cases, PGC1α expression or activity is increased by 80% in a treated stromal stem cell compared to an untreated stromal stem cell. In some cases, PGC1α expression or activity is increased by 100% in a treated stromal stem cell compared to an untreated stromal stem cell. In some cases, PGC1α expression or activity is increased by 150% in a treated stromal stem cell compared to an untreated stromal stem cell. In some cases, PGC1α expression or activity is increased by 200% in a treated stromal stem cell compared to an untreated stromal stem cell. In some cases, PGC1α expression or activity is increased by 250% in a treated stromal stem cell compared to an untreated stromal stem cell. In some cases, PGC1α expression or activity is increased by 300% in a treated stromal stem cell compared to an untreated stromal stem cell. In some cases, PGC1α expression or activity is increased by 350% in a treated stromal stem cell compared to an untreated stromal stem cell. In some cases, PGC1α expression or activity is increased by 400% in a treated stromal stem cell compared to an untreated stromal stem cell. In some cases, PGC1α expression or activity is increased by 450% in a treated stromal stem cell compared to an untreated stromal stem cell. In some cases, PGC1α expression or activity is increased by 500% in a treated stromal stem cell compared to an untreated stromal stem cell.

In certain aspects, provided herein are methods of preparing compositions for increasing monocyte and macrophage polarization to M2 phenotype comprising an exosome the method comprising treating a stromal stem cell to decrease expression of or inhibit CDC50. In some cases, the exosome is an SDC2+ exosome. In some cases, the method comprises isolating the exosome from a stromal stem cell treated with a CDC50 siRNA. In some cases, the method comprises isolating the exosome from a stromal stem cell treated with a virus expressing an shRNA targeting CDC50. In some cases, the method comprises isolating the exosome from a stromal stem cell treated with a CDC50 inhibitor.

In certain aspects, provided herein are methods for preparing compositions for increasing monocyte and macrophage polarization to M2 phenotype comprising an exosome treated to increase expression of or activate IRG1/ACOD1. In some cases, the exosome is an SDC2+ exosome. In some cases, the exosome is isolated from a stromal stem cell is treated with a IRG1/ACOD1 plasmid. In some cases, the exosome is isolated from a stromal stem cell is treated with a virus IRG1/ACOD1 IRG1/ACOD1. In some cases, the exosome is isolated from a stromal stem cell is treated with a PGC1α agonist.

In certain aspects, provided herein are methods for preparing compositions for increasing monocyte and macrophage polarization to M2 phenotype comprising an exosome treated to decrease expression of or inhibit CD47. In some cases, the exosome is an SDC2+ exosome. In some cases, the exosome is isolated from a stromal stem cell is treated with a CD47 siRNA. In some cases, the exosome is isolated from a stromal stem cell is treated with a virus expressing an shRNA targeting CD47. In some cases, the exosome is isolated from a stromal stem cell is treated with a CD47 inhibitor.

In certain aspects, provided herein are methods for preparing compositions for increasing monocyte and macrophage polarization to M2 phenotype comprising an exosome treated to increase expression of or activate PGC1α. In some cases, the exosome is an SDC2+ exosome. In some cases, the exosome is isolated from a stromal stem cell is treated with a PGC1α plasmid. In some cases, the exosome is isolated from a stromal stem cell is treated with a virus expressing PGC1α. In some cases, the exosome is isolated from a stromal stem cell is treated with a PGC1α agonist.

### Methods of Monocyte/Macrophage Polarization

Provided herein are methods of increasing monocyte and macrophage polarization to M2 phenotype comprising administering compositions comprising stromal stem cells and exosome compositions having increased capabilities of monocyte and macrophage polarization to M2 phenotype, such as SDC2+ stromal stem cells and SDC2+ exosomes treated to have at least one of increased mitochondrial numbers, increased mitochondrial density, increased cell surface phosphatidylserine, and increased itaconate compared to untreated SDC2+ stromal stem cells and SDC2+ exosomes. In some cases, stromal stem cells and exosomes for methods herein have increased capacity of transferring mitochondria to a target cell.

Methods of increasing monocyte and macrophage polarization to M2 phenotype in an individual provided herein, in certain aspects, comprise intracellularly administering mitochondria to cells of the individual. In some cases, methods of mitochondrial transfer to an individual comprise contacting cells of the individual to a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, stromal stem cells for methods of mitochondrial transfer herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Methods of increasing monocyte and macrophage polarization to M2 phenotype in an individual provided herein, in certain aspects, comprise intracellularly administering mitochondria to cells of the individual. In some cases, methods of mitochondrial transfer to an individual comprise contacting cells of the individual to an exosome isolated from a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, exosomes for methods of mitochondrial transfer herein are isolated from stromal stem cells having one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

In methods of increasing monocyte and macrophage polarization to M2 phenotype in an individual and methods of treatment provided herein, increasing monocyte and macrophage polarization to M2 phenotype in cells from the individual improves a symptom of a disease. In some cases, the individual has at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

### Methods of Mitochondrial Transfer

Provided herein are methods of mitochondrial transfer comprising administering compositions comprising stromal stem cells and exosome compositions having increased capabilities of mitochondrial transfer, such as SDC2+ stromal stem cells and SDC2+ exosomes treated to have at least one of increased mitochondrial numbers, increased mitochondrial density, increased cell surface phosphatidylserine, and increased itaconate compared to untreated SDC2+ stromal stem cells and SDC2+ exosomes. In some cases, stromal stem cells and exosomes for methods herein have increased capacity of transferring mitochondria to a target cell.

Methods of mitochondrial transfer to an individual provided herein, in certain aspects, comprise intracellularly administering mitochondria to cells of the individual. In some cases, methods of mitochondrial transfer to an individual comprise contacting cells of the individual to a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, stromal stem cells for methods of mitochondrial transfer herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Methods of mitochondrial transfer to an individual provided herein, in certain aspects, comprise intracellularly administering mitochondria to cells of the individual. In some cases, methods of mitochondrial transfer to an individual comprise contacting cells of the individual to an exosome isolated from a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, exosomes for methods of mitochondrial transfer herein are isolated from stromal stem cells having one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further provided herein are methods of treating a disease in an individual comprising administering to the individual a population of mammalian stromal stem cells, wherein the population of stromal stem cells has reduced expression of CDC50. Alternatively or in combination, the population of mammalian stromal stem cells has increased IRG1/ACOD1 expression or activity. In some cases, the population of mammalian stromal stem cells has reduced CD47 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Additionally provided herein are methods of treating a disease in an individual comprising administering to the individual a population of mammalian stromal stem cells, wherein the population of stromal stem cells has reduced expression of CD47. In some cases, the population of mammalian stromal stem cells has reduced CDC50 expression or activity. Alternatively or in combination, the population of mammalian stromal stem cells has increased IRG1/ACOD1 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further provided herein are methods of treating a disease in an individual comprising administering to the individual a population of mammalian stromal stem cells, wherein the population of stromal stem cells has increased expression of PGC1α. In some cases, the population of mammalian stromal stem cells has reduced CD47 expression or activity. Alternatively or in combination, the population of mammalian stromal stem cells has reduced CDC50 expression or activity. Alternatively or in combination, the population of mammalian stromal stem cells has increased IRG1/ACOD1 expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further provided herein are methods of treating a disease in an individual comprising administering to the individual exosomes isolated from a population of mammalian stromal stem cells, wherein the population of stromal stem cells has reduced expression of CDC50. Alternatively or in combination, the population of mammalian stromal stem cells has increased IRG1/ACOD1 expression or activity. In some cases, the population of mammalian stromal stem cells has reduced CD47 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Additionally provided herein are methods of treating a disease in an individual comprising administering to the individual exosomes isolated from a population of mammalian stromal stem cells, wherein the population of stromal stem cells has reduced expression of CD47. In some cases, the population of mammalian stromal stem cells has reduced CDC50 expression or activity. Alternatively or in combination, the population of mammalian stromal stem cells has increased IRG1/ACOD1 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further provided herein are methods of treating a disease in an individual comprising administering to the individual exosomes isolated from a population of mammalian stromal stem cells, wherein the population of stromal stem cells has increased expression of PGC1α. Alternatively or in combination, the population of mammalian stromal stem cells has reduced CDC50 expression or activity. Alternatively or in combination, the population of mammalian stromal stem cells has reduced IRG1/ACOD1 expression or activity. In some cases, the population of mammalian stromal stem cells has reduced CD47 expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

**In** methods of mitochondrial transfer to an individual and methods of treatment provided herein, transfer of mitochondria to cells from the individual improves a symptom of a disease. In some cases, the individual has at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

### Example 1: Preparation of Improved SDC2+ Stromal Stem Cells

Stromal stem cells are isolated from human umbilical cord tissue. The cells are treated with a transgene expressing a shRNA for downregulating CDC50 and CD47 and a second transgene expressing PGC1α. The treated cells are then sorted for cells expressing SDC2. A second population of stromal stem cells was treated with empty vector and sorted for cells expressing SDC2. The treated stromal stem cells are observed to have increased mitochondrial numbers as well as increased phosphatidylserine (PS) on the surface of the cell compared to the stromal stem cells treated with empty vector. Both populations of stromal stem cells are also observed to have standardized proliferative capacity.

This example shows that SDC2+ stromal stem cells having decreased CDC50 and CD47 and increased PGC1α have increased mitochondrial numbers and increased cell surface PS than control untreated SDC2+ stromal stem cells.

### Example 2: Preparation of Improved SDC2+ Exosomes

Stromal stem cells are isolated from human umbilical cord tissue. The cells are treated with a transgene expressing a shRNA for downregulating CDC50 and CD47 and a second transgene expressing PGC1α. Exosomes from the treated cells are then sorted for exosomes having SDC2. A second population of stromal stem cells was treated with empty vector and exosomes expressing SDC2 are isolated from the stromal stem cells. Exosomes from the treated stromal stem cells are observed to have increased mitochondrial numbers as well as increased phosphatidylserine (PS) on the surface of the exosome compared to exosomes from the stromal stem cells treated with empty vector.

This example shows that exosomes from SDC2+ stromal stem cells having decreased CDC50 and CD47 and increased PGC1α have increased mitochondrial numbers and increased cell surface PS than control exosomes from control untreated SDC2+ stromal stem cells.

### Example 3: In Vitro Mitochondrial Transfer with SDC2+ Stromal Stem Cells

Stromal stem cells are isolated from human umbilical cord tissue. The cells are treated with a transgene expressing a shRNA for downregulating CDC50 and CD47 and a second transgene expressing PGC1α. The treated cells are then sorted for cells expressing SDC2. A second population of stromal stem cells was treated with empty vector and sorted for cells expressing SDC2. The treated stromal stem cells are observed to have increased mitochondrial numbers as well as increased phosphatidylserine (PS) on the surface of the cell compared to the stromal stem cells treated with empty vector. Both populations of stromal stem cells are also observed to have standardized proliferative capacity.

The treated and sorted stromal stem cells are cultured with macrophages. The second population of empty vector treated, sorted stromal stem cells are cultured with a second population of macrophages. Both populations of macrophages are then stained for mitochondrial markers at various time points. The numbers of mitochondria in the macrophages cultured with treated stromal stem cells are observed to increase over time compared with control macrophages cultured in medium only. The numbers of mitochondria in the macrophages cultured with stromal stem cells that are treated with empty vector have an increase in mitochondria over time compared with control macrophages but the effect is reduced compared to macrophages cultured with treated stromal stem cells.

This example shows that SDC2+ stromal stem cells having decreased CDC50 and CD47 and increased PGC1α have increased efficacy in transferring mitochondria to treated cells than control SDC2+ stromal stem cells.

### Example 4: In Vitro Mitochondrial Transfer with SDC2+ Exosomes

Stromal stem cells are isolated from human umbilical cord tissue. The cells are treated with a transgene expressing a shRNA for downregulating CDC50 and CD47 and a second transgene expressing PGC1α. Exosomes from the treated cells are then sorted for exosomes having SDC2. A second population of stromal stem cells was treated with empty vector and exosomes expressing SDC2 are isolated from the stromal stem cells. Exosomes from the treated stromal stem cells are observed to have increased mitochondrial numbers as well as increased phosphatidylserine (PS) on the surface of the exosome compared to exosomes from the stromal stem cells treated with empty vector.

Exosomes from the treated and sorted stromal stem cells are cultured with macrophages. The second population of exosomes from empty vector treated, sorted stromal stem cells are cultured with a second population of macrophages. Both populations of macrophages are then stained for mitochondrial markers at various time points. The numbers of mitochondria in the macrophages cultured with exosomes from treated stromal stem cells are observed to increase over time compared with control macrophages cultured in medium only. The numbers of mitochondria in the macrophages cultured with exosomes from stromal stem cells that are treated with empty vector have an increase in mitochondria over time compared with control macrophages but the effect is reduced compared to macrophages cultured with exosomes from treated stromal stem cells.

This example shows that exosomes from SDC2+ stromal stem cells having decreased CDC50 and CD47 and increased PGC1α have increased efficacy in transferring mitochondria to treated cells than control exosomes from SDC2+ stromal stem cells.

### Example 5: Treatment of Diabetic Ulcers

A composition comprising SDC2+ stromal stem cells that have been treated with siRNA to reduce CDC50 and CD47 and a PGC1α transgene formulated in a collagen matrix is used to treat a group of individuals in need of treatment of diabetic ulcers. After administration of the treated SDC2+ stromal stem cell composition and covering the ulcers with a bandage, the diabetic ulcers are shown to fully heal in 10/10 treated individuals.

A second population of individuals needing treatment for diabetic ulcers is treated with a composition comprising SDC2+ stromal stem cells treated with an empty vector. After administration of the empty-vector treated SDC2+ stromal stem cells and covering the ulcers with a bandage, the diabetic ulcers are shown to fully heal in 7/10 of the treated individuals and decrease in severity in 9/10 of the treated individuals.

This example shows that SDC2+ stromal stem cells having decreased CDC50 and CD47 and increased PGC1α have increased efficacy in treating diabetic ulcers than control SDC2+ stromal stem cells.

### Example 6: Treatment of Diabetic Ulcers

A composition comprising exosomes from SDC2+ stromal stem cells that have been treated with siRNA to reduce CDC50 and CD47 and a PGC1α transgene formulated in a collagen matrix is used to treat a group of individuals in need of treatment of diabetic ulcers. After administration of exosomes from the treated SDC2+ stromal stem cell composition and covering the ulcers with a bandage, the diabetic ulcers are shown to fully heal in 10/10 treated individuals.

A second population of individuals needing treatment for diabetic ulcers is treated with a composition comprising exosomes from SDC2+ stromal stem cells treated with an empty vector. After administration of exosomes from the empty-vector treated SDC2+ stromal stem cells and covering the ulcers with a bandage, the diabetic ulcers are shown to fully heal in 7/10 of the treated individuals and decrease in severity in 9/10 of the treated individuals.

This example shows that exosomes from SDC2+ stromal stem cells having decreased CDC50 and CD47 and increased PGC1α have increased efficacy in treating diabetic ulcers than exosomes from control SDC2+ stromal stem cells.

### Example 7: Treatment of Inflammatory Liver Disease

A composition comprising SDC2+ stromal stem cells that have been treated with siRNA to reduce CDC50 and CD47 and a PGC1α transgene formulated for intravenous administration is used to treat a group of individuals in need of treatment of primary sclerosing cholangitis or autoimmune hepatitis. After administration of the treated SDC2+ stromal stem cell composition, serum inflammatory markers are reduced and liver function markers are increased 10/10 treated individuals.

A second population of individuals needing treatment for primary sclerosing cholangitis or autoimmune hepatitis is treated with a composition comprising SDC2+ stromal stem cells treated with an empty vector. After administration of the empty-vector treated SDC2+ stromal stem cells, serum inflammatory markers are reduced and liver function markers are increased in 7/10 of the treated individuals.

This example shows that SDC2+ stromal stem cells having decreased CDC50 and CD47 and increased PGC1α have increased efficacy in treating primary sclerosing cholangitis or autoimmune hepatitis than control SDC2+ stromal stem cells.

### Example 8: Treatment of Inflammatory Liver Disease

A composition comprising exosomes from SDC2+ stromal stem cells that have been treated with siRNA to reduce CDC50 and CD47 and a PGC1α transgene formulated for intravenous administration is used to treat a group of individuals in need of treatment of primary sclerosing cholangitis or autoimmune hepatitis. After administration of exosomes from the treated SDC2+ stromal stem cell composition, serum inflammatory markers are reduced and liver function markers are increased in 10/10 treated individuals.

A second population of individuals needing treatment for primary sclerosing cholangitis or autoimmune hepatitis is treated with a composition comprising exosomes from SDC2+ stromal stem cells treated with an empty vector. After administration of exosomes from the empty-vector treated SDC2+ stromal stem cells, inflammatory markers are reduced and liver function markers are increased in 7/10 of the treated individuals.

This example shows that exosomes from SDC2+ stromal stem cells having decreased CDC50 and CD47 and increased PGC1α have increased efficacy in treating primary sclerosing cholangitis or autoimmune hepatitis than exosomes from control SDC2+ stromal stem cells.

### Example 9: Treatment of Diabetic Kidney Disease

A composition comprising SDC2+ stromal stem cells that have been treated with siRNA to reduce CDC50 and CD47 and a PGC1α transgene formulated for intravenous administration is used to treat a group of individuals in need of treatment of diabetic kidney disease. After administration of the treated SDC2+ stromal stem cell composition, serum inflammatory markers are reduced and kidney function markers are increased 10/10 treated individuals.

A second population of individuals needing treatment for diabetic kidney disease is treated with a composition comprising SDC2+ stromal stem cells treated with an empty vector. After administration of the empty-vector treated SDC2+ stromal stem cells, serum inflammatory markers are reduced and kidney function markers are increased in 7/10 of the treated individuals.

This example shows that SDC2+ stromal stem cells having decreased CDC50 and CD47 and increased PGC1α have increased efficacy in treating diabetic kidney disease than control SDC2+ stromal stem cells.

### Example 10: Treatment of Diabetic Kidney Disease

A composition comprising exosomes from SDC2+ stromal stem cells that have been treated with siRNA to reduce CDC50 and CD47 and a PGC1α transgene formulated for intravenous administration is used to treat a group of individuals in need of treatment of diabetic kidney disease. After administration of exosomes from the treated SDC2+ stromal stem cell composition, serum inflammatory markers are reduced and kidney function markers are increased in 10/10 treated individuals.

A second population of individuals needing treatment for diabetic kidney disease is treated with a composition comprising exosomes from SDC2+ stromal stem cells treated with an empty vector. After administration of exosomes from the empty-vector treated SDC2+ stromal stem cells, inflammatory markers are reduced and kidney function markers are increased in 7/10 of the treated individuals.

This example shows that exosomes from SDC2+ stromal stem cells having decreased CDC50 and CD47 and increased PGC1α have increased efficacy in treating diabetic kidney disease than exosomes from control SDC2+ stromal stem cells.

### Example 11: Treatment of Chronic Obstructive Pulmonary Disease

A composition comprising SDC2+ stromal stem cells that have been treated with siRNA to reduce CDC50 and CD47 and a PGC1α transgene formulated for pulmonary administration is used to treat a group of individuals in need of treatment of chronic obstructive pulmonary disease. After administration of the treated SDC2+ stromal stem cell composition, serum inflammatory markers are reduced and lung function is increased 10/10 treated individuals.

A second population of individuals needing treatment for chronic obstructive pulmonary disease is treated with a composition comprising SDC2+ stromal stem cells treated with an empty vector. After administration of the empty-vector treated SDC2+ stromal stem cells, serum inflammatory markers are reduced and lung function is increased in 7/10 of the treated individuals.

This example shows that SDC2+ stromal stem cells having decreased CDC50 and CD47 and increased PGC1α have increased efficacy in treating chronic obstructive pulmonary disease than control SDC2+ stromal stem cells.

### Example 12: Treatment of Chronic Obstructive Pulmonary Disease

A composition comprising exosomes from SDC2+ stromal stem cells that have been treated with siRNA to reduce CDC50 and CD47 and a PGC1α transgene formulated for pulmonary administration is used to treat a group of individuals in need of treatment of chronic obstructive pulmonary disease. After administration of exosomes from the treated SDC2+ stromal stem cell composition, serum inflammatory markers are reduced and lung function is increased in 10/10 treated individuals.

A second population of individuals needing treatment for diabetic kidney disease is treated with a composition comprising exosomes from SDC2+ stromal stem cells treated with an empty vector. After administration of exosomes from the empty-vector treated SDC2+ stromal stem cells, inflammatory markers are reduced and lung function is increased in 7/10 of the treated individuals.

This example shows that exosomes from SDC2+ stromal stem cells having decreased CDC50 and CD47 and increased PGC1α have increased efficacy in treating chronic obstructive pulmonary disease than exosomes from control SDC2+ stromal stem cells.

### Example 13: Correlation between the amount of indoleamine 2,3-dioxygenase (IDO) produced and the T cell inhibitory potential of MSC donors

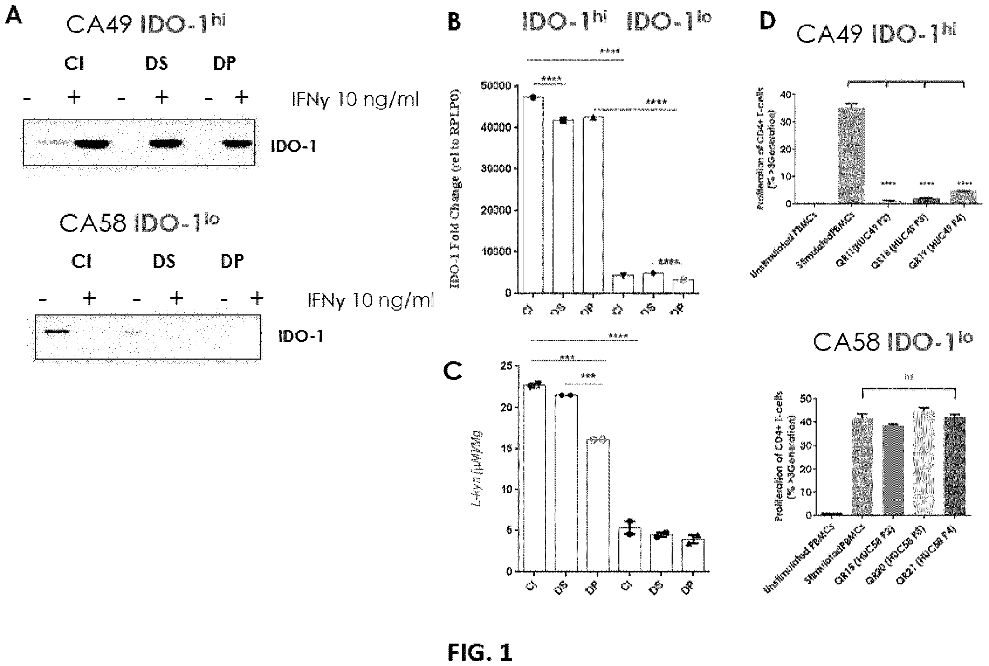

MSCs (CA49 and CA58) were cultured for 24 h, stimulated for 24 h and harvested before total cell lysates were assayed for human IDO. The expression levels of human IDO protein were determined by western blotting analysis as shown in **Fig. 1A****.** Unstimulated MSCs served as a negative control, and human MSCs stimulated with the inflammatory cytokine IFNΥ (10 ng/mL) served as positive control. As shown, IDO-1^{hi} DP retains IDO-1 expression.

IDO-1^{hi} (CA49DP) and IDO-1^{lo} (CA58DP) quantum-expanded donors were stimulated with 10 ng/mL for 24 h. The expression level of IDO mRNA relative to RPLP0 was evaluated by Taqman real-time QPCR as shown in **Fig. 1B****.** The Delta-delta CT method was applied to calculate the fold induction and error bars indicate upper and lower limit. Control IDO-1 gene expression following IFN 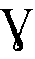 (10 ng/ml, 24 h) in an IDO-1hi and IDO-1lo donor.

**Fig. 1C** shows an impact of IFN 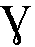 on the enzymatic activity of IDO harvested from conditioned media of IDO-1hi and IDO-1lo donors measured by production of kynurenine. As shown, IDO-1^{hi} DP retains the enzymatic activity of IDO compared to IDO-1^{lo.}

**Fig. 1D** shows bar graphs of T cell proliferation assay. T cell proliferation was measured in a proliferation assay performed with carboxyfluorescein diacetate succinimidyl ester (CFSE)-labeled PBMC activated with anti-CD3/CD28 antibodies in the presence of either IDO-1^{hi} or IDO-1^{lo} MSC donors. As shown, IDO-1^{hi} DP suppresses PBMC proliferation.

### Example 14: IDO-1^{hi} DP donor is capable of inducing immune regulatory monocytes in comparison to IDO-1^{lo}DP donor in a 24hr monocyte assay

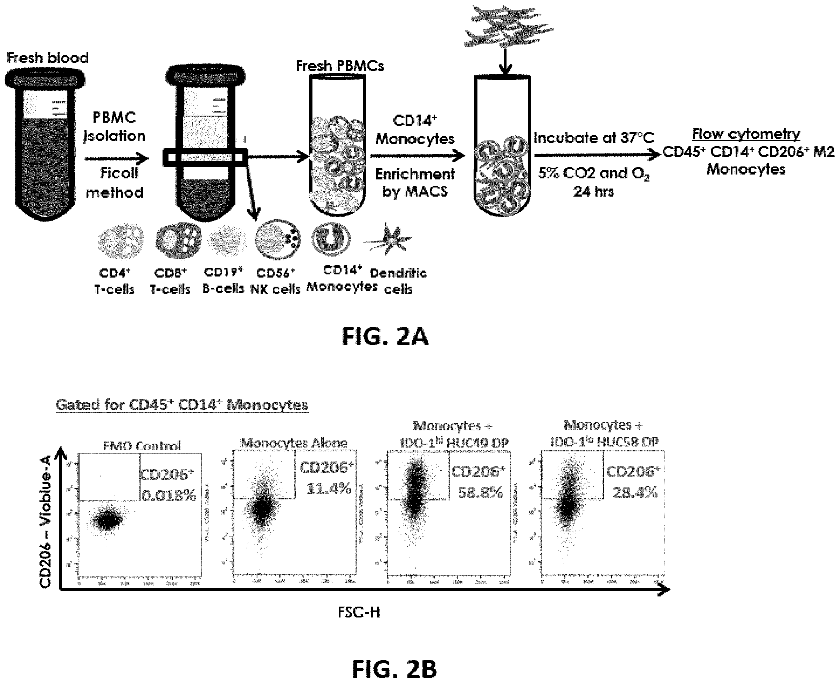

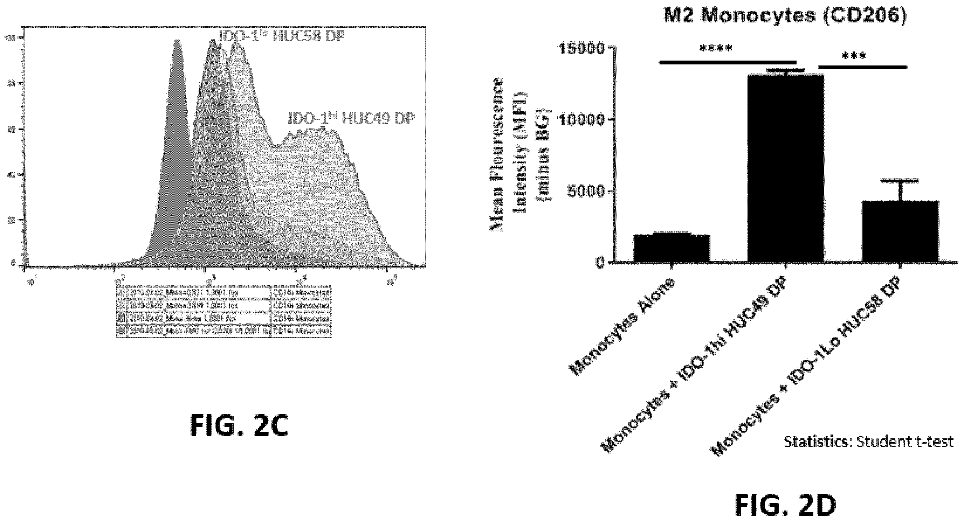

MSCs (ORBCEL-C) were cultured with freshly MACS isolated CD14⁺ monocytes for 24hrs as shown in the schematic diagram of **Fig. 2A****.** In 24 hrs post co-culture of MSC and monocytes, cells were labelled & gated for CD45⁺ CD14⁺ CD206⁺ M2 monocytes. **Fig. 2B** shows FACS assay results of CD206+ populations (control, Monocytes alone, Monocytes + IDO-1^{hi} HUC49 DP (ORBCEL-C), and Monocytes + IDO-1^{lo} HUC58 DP (ORBCEL-C) from left to right). As shown, in Fig. 2C in the histogram depicting the expression of CD206 on cells gated for monocyte population (CD45⁺CD14⁺), CD206+ cell population was increased with co-culture of Monocytes + IDO-1^{hi} HUC49 DP (ORBCEL-C) compared to other groups. Fig. 2D shows representative bar graph showing Mean Fluorescence intensity (MFI) of CD206 expression on monocytes between different conditions (n=4). Statistics: Students T-test.

### Example 15: Reduction in TMEM30A expression results in significantly increased PtS on the cell surface of ORBCEL-C

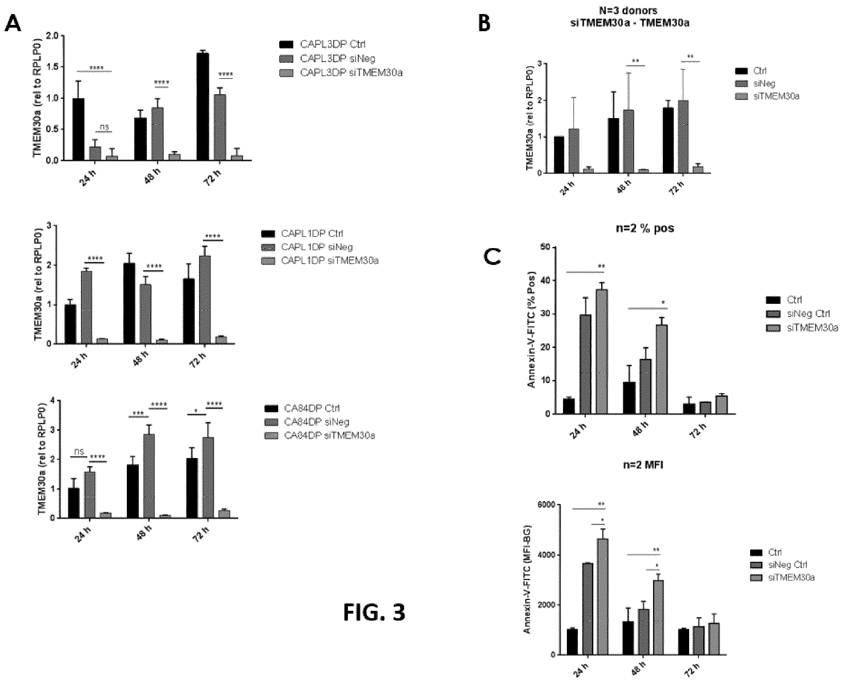

Three independent donor pools of ORBCEL-C were transfected with siRNA directed at TMEM30A or negative siRNA. The expression level of TMEM30A relative to RPLP0 was evaluated by Taqman real-time qPCR. As shown in **Fig. 3A****,** introduction of siRNA directed at TMEM30A significantly reduced the expression of TMEM30A in all three independent donor pools of ORBCEL-C. The Delta-delta CT method was applied to calculate the fold induction and error bars indicates upper and lower limit. **Fig. 3B** shows a combined graph of TMEM30A gene expression of the three pooled ORBCEL-C. **Fig. 3C** shows a graph of flow cytometry (% Pos and MFI) of WT, siTMEM30A and siNegative control cells labelled with annexin V-FITC. As shown, expression of surface phosphatidylserine (PtS) in ORBCEL-C was increased by 48 hours with reduction of TMEM30A expression by siRNA directed at TMEM30A.

### Example 16: Impact of siTMEM30A on ORBCEL-C phenotype

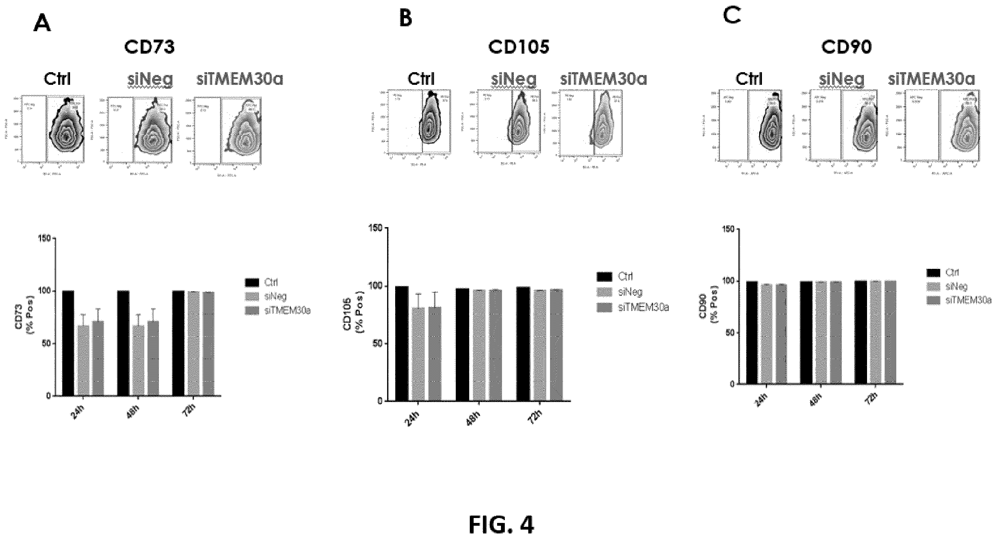

MSCs were plated at 2×10⁵ cells per well in a 6-well plate. Following 24 h cells were transfected with siRNA directed at TMEM30A or negative siRNA and untransfected as controls. Cells were harvested and analysed at 24, 48 and 72 h. A, B and C show % positive for each condition. (Bars are mean ±SEM, One-way ANOVA, n=3. The phenotype of ORBCEL-C^{™} cells were determined by flow cytometric analysis of expression levels of CD73, CD105, and CD90 in the MSCs. As shown in **Figs. 4A-**C, expression levels of CD73, CD105, and CD90 in the MSCs did not change substantially by inhibiting TMEM30A expression.

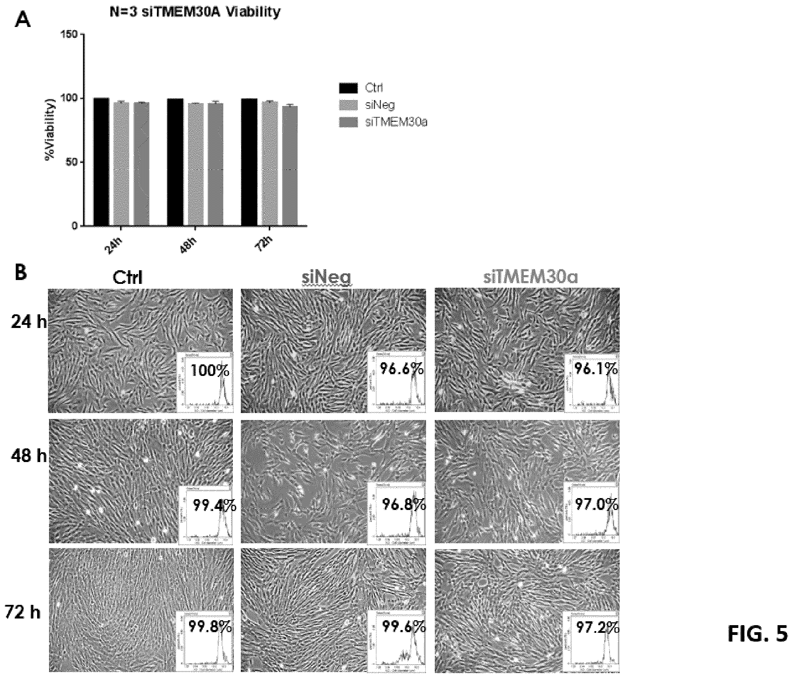

Also, at 24, 48 and 72 hours after transfection, cells were harvested and viability was measured by both NC200 (acridine orange and DAPI stain) and staining with Sytox blue and analyzing by flow cytometry. As shown in **Fig. 5A****,** cell viability of ORBCEL-C was not affected in 24 h, 48h, or 72h after the treatment with siRNA directed at TMEM30A. **Fig. 5B** shows exemplary and representative phase contrast images of MSCs transfected with siTMEM30A that show no change in morphology, density, or adherence.

### Example 17: Reduction in TMEM30A expression and increased PtS results in higher IDO-1 expression (converts IDO-1^{lo} to IDO-1^{hi})

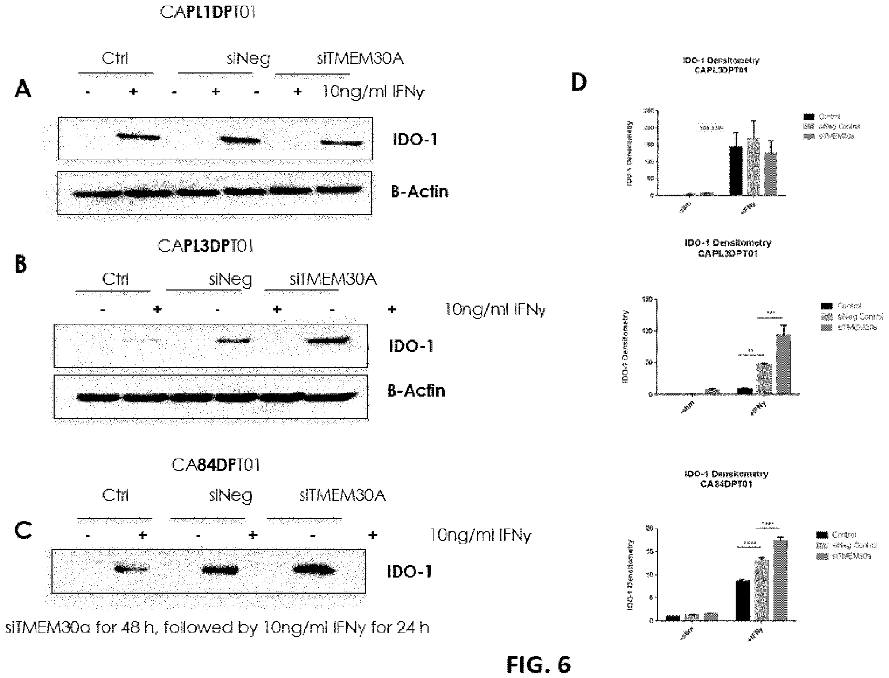

Three independent donor pools of ORBCEL-C^{™} (CAPL1DP **(****Fig. 6A****),** CAPL3DP **(****Fig. 6B****),** C CA84DP **(****Fig. 6C****))** were transfected with siRNA directed at TMEM30A or negative siRNA. Human IDO protein was determined by western blotting analysis. MSCs were cultured for 24 h, transfected with siTMEM30A/siNeg Ctrl for 48 h followed by stimulation for 24 h and harvested before total cell lysates were assayed for human IDO. Unstimulated MSCs served as a negative control; human MSCs stimulated with the inflammatory cytokine IFN 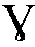 (10 ng/mL) served as positive control. As shown in **Fig. 6D** (densitometry analysis of western blots shown in **Figs. 6A-C**expression of IDO-1 was significantly increased in CAPL3DP and CA84DP cells upon treatment with siRNA directed at TMEM30A.

### Example 18: TMEM30A knowndown (KD) Switches IDO-1^{lo} donor's M2 monocytes induction from Low to High

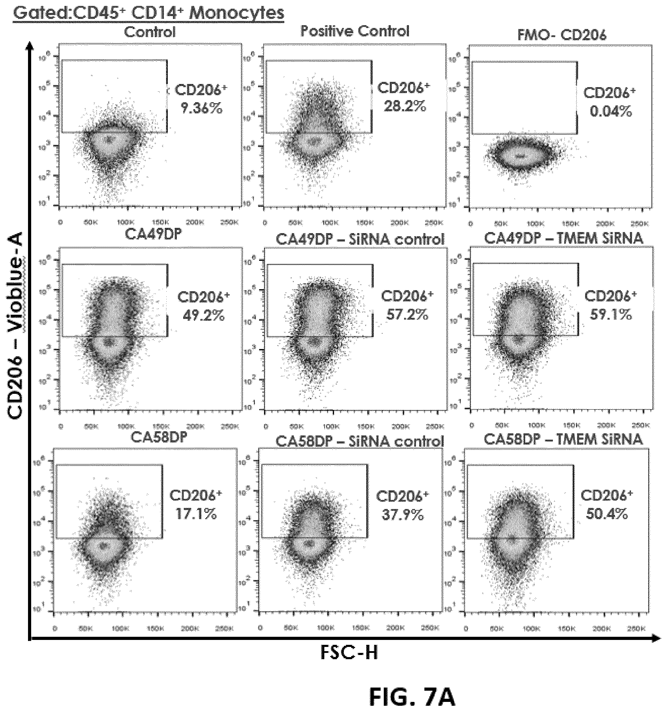

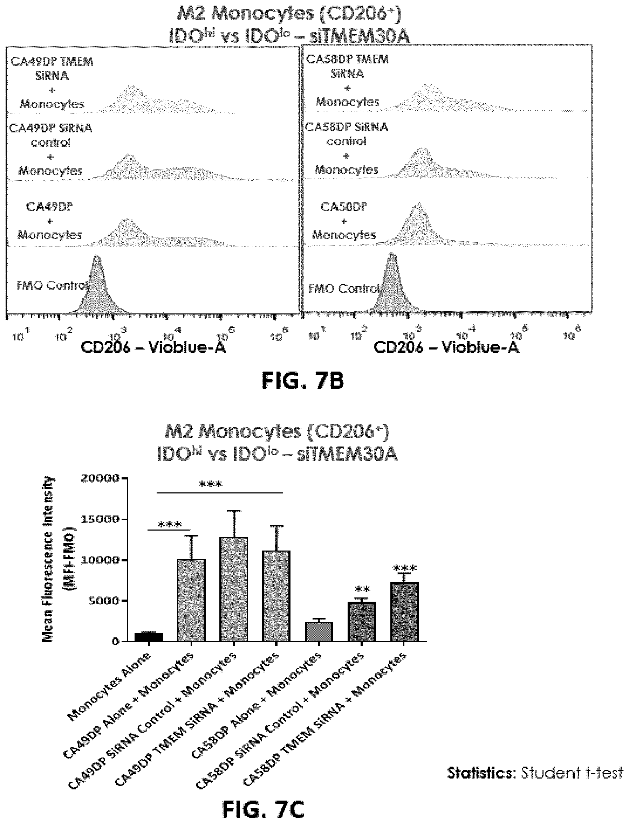

MSCs (ORBCEL-C) either TMEM30A siRNA knockdown MSCs or controls siRNA MSCs were cultured with freshly MACS isolated CD14⁺ monocytes for 24hrs. 24hrs post co-culture, cells were labelled & gated for CD45⁺ CD14⁺ CD206⁺ M2 monocytes. **Fig. 7A** shows FACS analysis of cell population in each condition: (Conditions - 1) Monocytes alone, 2) Monocytes + IDO-1^{hi} HUC49 DP (ORBCEL-C), 3) Monocytes + IDO-1^{lo} HUC58 DP. 4) Monocytes + IDO-1^{hi} HUC49 DP SiRNA control, 5) Monocytes + IDO-1^{hi} HUC49 DP TMEM SiRNA, 6) Monocytes + IDO-1^{lo} HUC58 siRNA control & 7) Monocytes + IDO-1^{lo} HUC58 TMEM SiRNA). **Fig. 7B** shows a histogram depicting the expression of CD206 on cells gated for monocyte population (CD45⁺CD14⁺) from different culture conditions. **Fig. 7C** shows a representative bar graph showing Mean Fluorescence intensity (MFI) of CD206 expression on monocytes between different culture conditions (n=1). Statistics: Students T-test. As shown, TMEM30A knockdown switches IDO-1^{lo} donor's M2 monocytes induction from Low to High (increased CD206+ cells) while TMEM30A knockdown does not significantly affect the IDO-1^{high} donor's M2 monocytes induction.

### Example 19: Induction of regulatory monocytes (CD163⁺ CD206⁺) by IDO-1^{hi} DP is via an active cell-cell interaction process

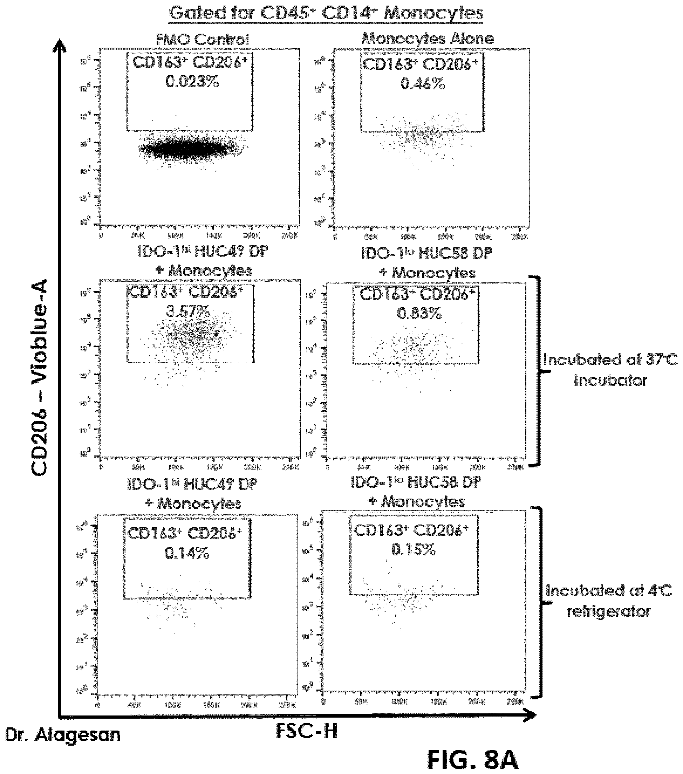

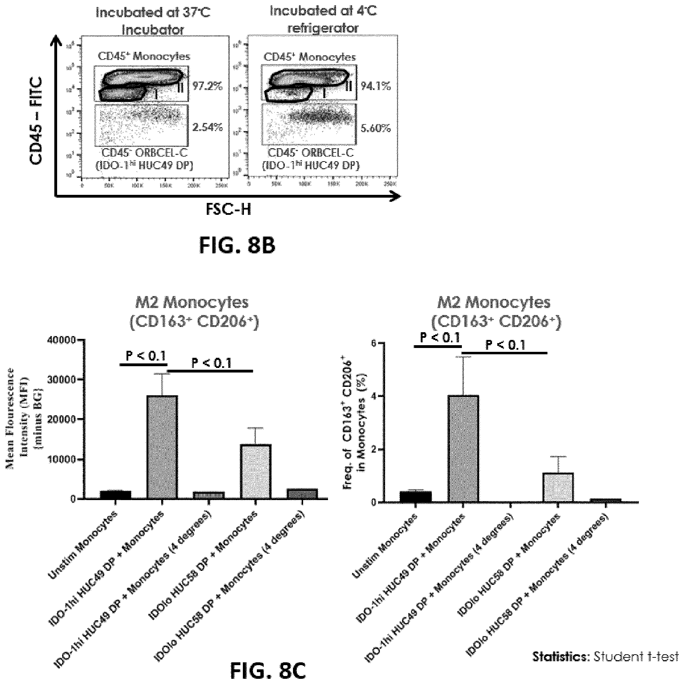

MSCs (ORBCEL-C) were cultured with freshly MACS isolated CD14⁺ monocytes for 24hrs either at 37°C incubator or at 4°C refrigerator. 24hrs post co-culture, cells were labelled & gated for CD45⁺ CD14⁺ CD163⁺ CD206⁺ M2 monocytes. **Fig. 8A** shows a FACS analysis of cell population in each condition: Conditions - 1) Monocytes alone, 2) Monocytes + IDO-1^{hi} HUC49 DP (ORBCEL-C) (37°C or 4°C) & 3) Monocytes + IDO-1^{lo} HUC58 DP (ORBCEL-C) (37°C or 4°C). **Fig 8B** shows flow cytometry dot plots showing two populations of CD45⁺ monocytes (I & II) modulated by MSCs (ORBCEL-C) and CD45⁻ IDO-1^{hi} HUC49 DP (ORBCEL-C) cultured either at 37°C or 4°C. Fig 8C shows Representative bar graph showing Mean Fluorescence intensity (MFI) of CD163⁺ CD206⁺ expression on monocytes & percentage of CD163⁺ CD206⁺ monocytes between different conditions (n=1). Statistics: Students T-test.

### Example 20: IDO-1^{hi} donor primed monocytes are capable of inducing regulatory T-cells (T-regs)

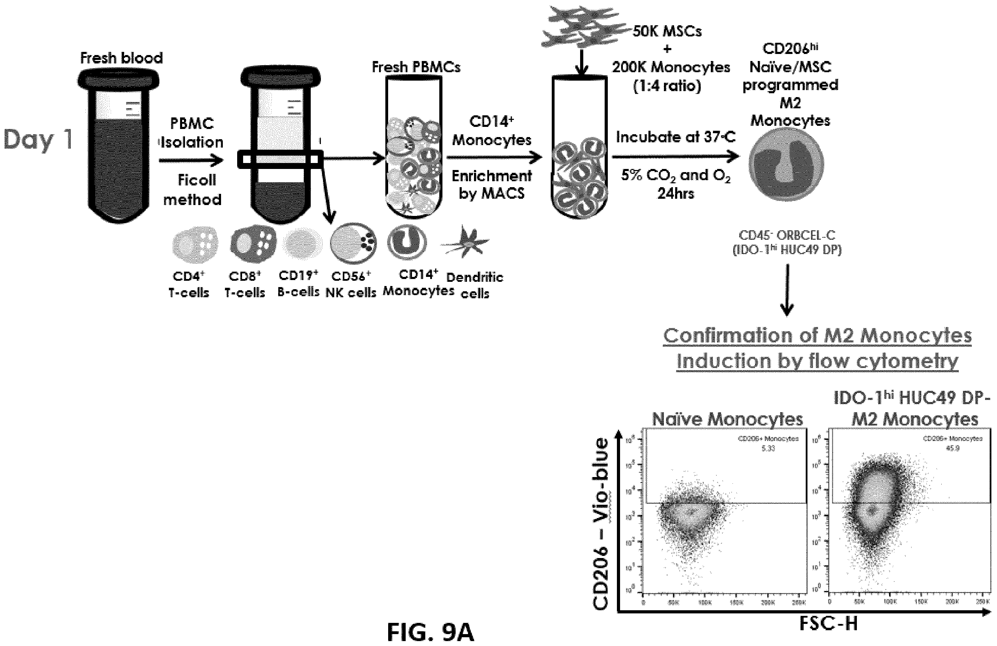

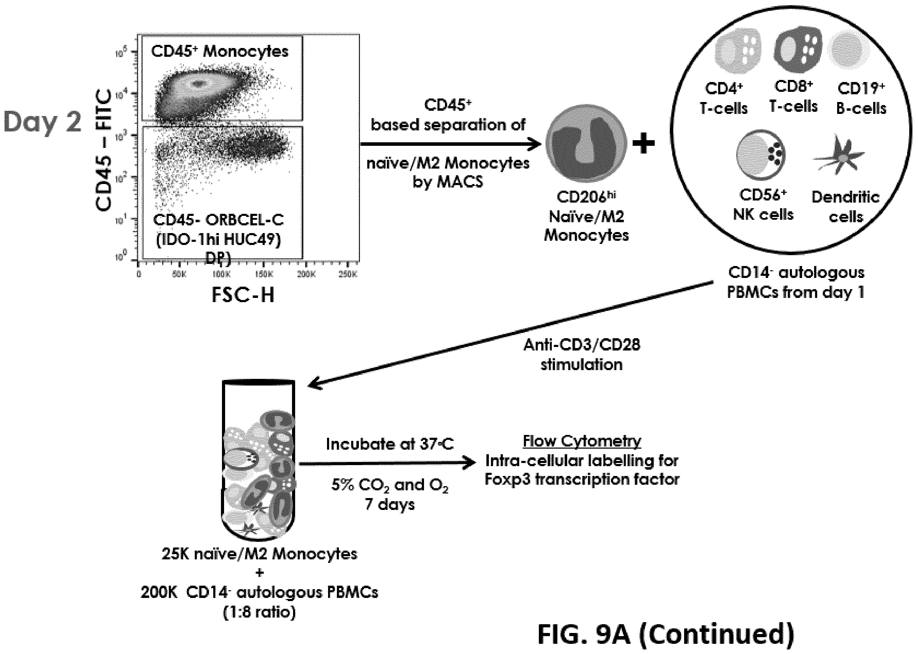

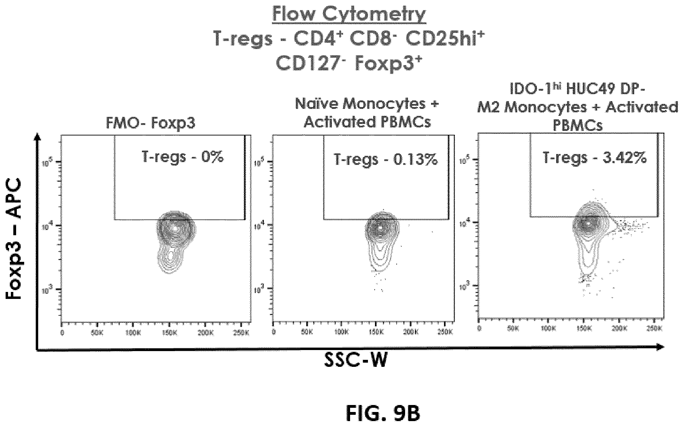

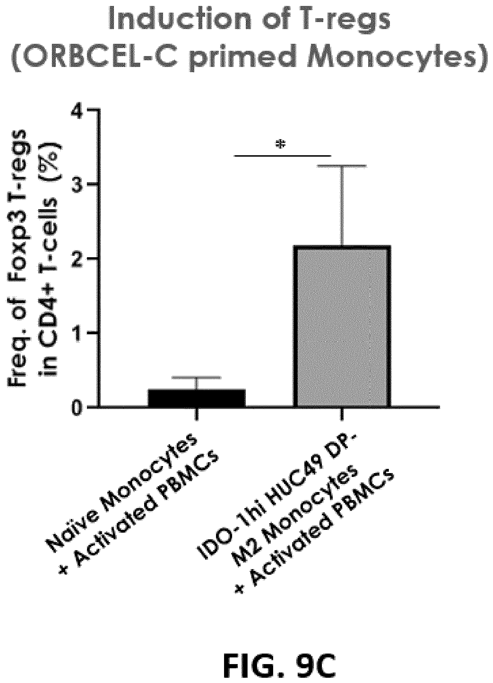

Monocytes and MSCs were co-cultured for 24 hrs. Following 24hrs co-culture of MSC, monocytes were purified by CD45⁺ MACS separation and cultured for 7 days with autologous CD3 CD28 activated PBMCs. **Fig. 9A** shows a schematic diagram explaining the co-culture of MSC primed monocytes: autologous PBMCs for T-reg assay. Regulatory T-cells were analysed by intra-cellular labelling of Foxp3 and gated for CD4⁺ CD8⁻ CD25hi⁺ CD127⁻ Foxp3⁺ regulatory T-cells. **Fig. 9B** shows flow cytometry analysis of CD4⁺ CD8⁻ CD25hi⁺ CD127⁻ Foxp3⁺ regulatory T-cell population in various conditions: Conditions - 1) Naive Monocytes + Activated PBMCs, and 2) IDO-1^{hi} HUC49 DP - M2 Monocytes + Activated PBMCs. Fig. 9C shows a representative bar graph showing percentage of regulatory T-cells in CD4⁺ T-cells from different culture conditions (n=1). Statistics: Students T-test. As shown, the frequency of Foxp3⁺ regulatory T-cells among the CD4+ T cells was significantly increased when the monocytes were primed with ORBCEL-C.

Subject matter of this application is also disclosed in the following paragraphs that describe and/or define embodiments of the invention:
1. A method of treating an inflammatory disease in an individual, the method comprising administering a population of stromal stem cells to the individual in an amount effective to reduce at least one symptom of the inflammatory disease, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47.
2. The method of embodiment 1, wherein the population of mammalian stromal stem cells has reduced CDC50 activity.
3. The method of embodiment 1 or embodiment 2, wherein the population of mammalian stromal stem cells has increased IRG1/ACOD1 activity.
4. The method of any one of embodiments 1 to 3, wherein the population of mammalian stromal stem cells has increased PGC1α activity.
5. The method of any one of embodiments 1 to 4, wherein the population of mammalian stromal stem cells has reduced CD47 activity.
6. The method of any one of embodiments 1 to 5, wherein the population of mammalian stromal stem cells is at least 30% positive for SDC2.
7. The method of any one of embodiments 1 to 6, wherein CDC50 activity is reduced by treating the population of mammalian stromal stem cells with at least one of a CDC50 antagonist, a CDC50 inhibitory nucleic acid, and a CDC50 inhibitory peptide.
8. The method of any one of embodiments 1 to 7, wherein IRG1/ACOD1 activity is increased by treating the population of mammalian stromal stem cells with at least one of an IRG1/ACOD1 agonist and a nucleic acid encoding IRG1/ACOD1.
9. The method of any one of embodiments 1 to 8, wherein PGC1α activity is increased by treating the population of mammalian stromal stem cells with at least one of a PGC1α agonist and a nucleic acid encoding PGC1α.
10. The method of any one of embodiments 1 to 9, wherein CD47 activity is reduced by treating the population of mammalian stromal stem cells with at least one of a CD47 antagonist, a CD47 inhibitory nucleic acid, and a CD47 inhibitory peptide.
11. The method of any one of embodiments 1 to 10, wherein the method increases the total number of mitochondria in the cell.
12. The method of any one of embodiments 1 to 11, wherein the method increases the total volume of mitochondria in the cell.
13. The method of any one of embodiments 1 to 12, wherein the method increases phosphatidylserine (PS) levels on the cell surface.
14. The method of any one of embodiments 1 to 13, wherein the method increases itaconate levels in the cell.
*15.* The method of any one of embodiments 1 to 14, wherein the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
16. A method of treating an individual, the method comprising administering to the individual a population of mammalian stromal stem cells, wherein the population of mammalian stromal stem cells has reduced CDC50 activity.
17. The method of embodiment 16, wherein the population of mammalian stromal stem cells has increased IRG1/ACOD1 activity.
18. The method of embodiment 16 or embodiment 17, wherein the population of mammalian stromal stem cells has increased PGC1α activity.
19. The method of any one of embodiments 16 to 18, wherein the population of mammalian stromal stem cells has reduced CD47 activity.
20. The method of any one of embodiments 16 to 19, wherein the population of mammalian stromal stem cells is at least 30% positive for SDC2.
21. The method of any one of embodiments 16 to 20, wherein CDC50 activity is reduced by treating the population of mammalian stromal stem cells with at least one of a CDC50 antagonist, a CDC50 inhibitory nucleic acid, and a CDC50 inhibitory peptide.
22. The method of any one of embodiments 17 to 21, wherein IRG1/ACOD1 activity is increased by treating the population of mammalian stromal stem cells with at least one of an IRG1/ACOD1 agonist and a nucleic acid encoding IRG1/ACOD1.
23. The method of any one of embodiments 18 to 22, wherein PGC1α activity is increased by treating the population of mammalian stromal stem cells with at least one of a PGC1α agonist and a nucleic acid encoding PGC1α.
24. The method of any one of embodiments 19 to 22, wherein CD47 activity is reduced by treating the population of mammalian stromal stem cells with at least one of a CD47 antagonist, a CD47 inhibitory nucleic acid, and a CD47 inhibitory peptide.
25. The method of any one of embodiments 16 to 24, wherein the method increases the total number of mitochondria in cells of the individual.
26. The method of any one of embodiments 16 to 25, wherein the method increases the total volume of mitochondria in cells of the individual.
27. The method of any one of embodiments 16 to 26, wherein the method increases phosphatidylserine (PS) levels on the cell surface.
28. The method of any one of embodiments 16 to 27, wherein the method increases itaconate levels in the cell.
29. The method of any one of embodiments 16 to 28, wherein the individual exhibits at least one symptom of disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
30. A method of treating an individual, the method comprising administering to the individual a population of mammalian stromal stem cells, wherein the population of mammalian stromal stem cells has increased IRG1/ACOD1 activity.
31. The method of embodiment 30, wherein the population of mammalian stromal stem cells has reduced CDC50 activity.
32. The method of embodiment 30 or embodiment 31, wherein the population of mammalian stromal stem cells has increased PGC1α activity.
33. The method of any one of embodiments 30 to 32, wherein the population of mammalian stromal stem cells has reduced CD47 activity.
34. The method of any one of embodiments 30 to 33, wherein the population of mammalian stromal stem cells is at least 30% positive for SDC2.
35. The method of any one of embodiments 31 to 34, wherein CDC50 activity is reduced by treating the population of mammalian stromal stem cells with at least one of a CDC50 antagonist, a CDC50 inhibitory nucleic acid, and a CDC50 inhibitory peptide.
36. The method of any one of embodiments 32 to 35, wherein PGC1α activity is increased by treating the population of mammalian stromal stem cells with at least one of a PGC1α agonist and a nucleic acid encoding PGC1α.
37. The method of any one of embodiments 33 to 36, wherein CD47 activity is reduced by treating the population of mammalian stromal stem cells with at least one of a CD47 antagonist, a CD47 inhibitory nucleic acid, and a CD47 inhibitory peptide.
38. The method of any one of embodiments 30 to 37, wherein the method increases the total number of mitochondria in cells of the individual.
39. The method of any one of embodiments 30 to 38, wherein the method increases the total volume of mitochondria in cells of the individual.
40. The method of any one of embodiments 30 to 39, wherein the method increases phosphatidylserine (PS) levels on the cell surface.
41. The method of any one of embodiments 30 to 40, wherein the method increases itaconate levels in the cell.
42. The method of any one of embodiments 30 to 41, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
43. A method of treating an individual, the method comprising administering to the individual a population of mammalian stromal stem cells, wherein the population of mammalian stromal stem cells has increased expression of PGC1α.
44. The method of embodiment 43, wherein the population of mammalian stromal stem cells has reduced CDC50 activity.
45. The method of embodiment 43 or embodiment 44, wherein the population of mammalian stromal stem cells has increased IRG1/ACOD1 activity.
46. The method of any one of embodiments 43 to 45, wherein the population of mammalian stromal stem cells has reduced CD47 activity.
47. The method of any one of embodiments 43 to 46, wherein the population of mammalian stromal stem cells is at least 30% positive for SDC2.
48. The method of any one of embodiments 43 to 47, wherein PGC1α activity is increased by treating the population of mammalian stromal stem cells with at least one of a PGC1α agonist and a nucleic acid encoding PGC1α.
49. The method of any one of embodiments 44 to 48, wherein CDC50 activity is reduced by treating the population of mammalian stromal stem cells with at least one of a CDC50 antagonist, a CDC50 inhibitory nucleic acid, and a CDC50 inhibitory peptide.
50. The method of any one of embodiments 45 to 49, wherein IRG1/ACOD1 activity is increased by treating the population of mammalian stromal stem cells with at least one of an IRG1/ACOD1 agonist and a nucleic acid encoding IRG1/ACOD1.
51. The method of any one of embodiments 46 to 50, wherein CD47 activity is reduced by treating the population of mammalian stromal stem cells with at least one of a CD47 antagonist, a CD47 inhibitory nucleic acid, and a CD47 inhibitory peptide.
52. The method of any one of embodiments 46 to 51, wherein the method increases the total number of mitochondria in cells of the individual.
53. The method of any one of embodiments 46 to 52, wherein the method increases the total volume of mitochondria in cells of the individual.
54. The method of any one of embodiments 30 to 39, wherein the method increases phosphatidylserine (PS) levels on the cell surface.
55. The method of any one of embodiments 30 to 40, wherein the method increases itaconate levels in the cell.
56. The method of any one of embodiments 46 to 53, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
57. A method of treating an individual, the method comprising administering to the individual a population of mammalian stromal stem cells, wherein the population of mammalian stromal stem cells has reduced expression of CD47.
58. The method of embodiment 57, wherein the population of mammalian stromal stem cells has reduced CDC50 activity.
59. The method of embodiment 57 or embodiment 58, wherein the population of mammalian stromal stem cells has increased IRG1/ACOD1 activity.
60. The method of any one of embodiments 57 to 59, wherein the population of mammalian stromal stem cells has increased PGC1α activity.
61. The method of any one of embodiments 57 to 60, wherein the population of mammalian stromal stem cells is at least 30% positive for SDC2.
62. The method of any one of embodiments 57 to 61, wherein CD47 activity is reduced by treating the population of mammalian stromal stem cells with at least one of a CD47 antagonist, a CD47 inhibitory nucleic acid, and a CD47 inhibitory peptide.
63. The method of any one of embodiments 58 to 62, wherein CDC50 activity is reduced by treating the population of mammalian stromal stem cells with at least one of a CDC50 antagonist, a CDC50 inhibitory nucleic acid, and a CDC50 inhibitory peptide.
64. The method of any one of embodiments 59 to 63, wherein IRG1/ACOD1 activity is increased by treating the population of mammalian stromal stem cells with at least one of an IRG1/ACOD1 agonist and a nucleic acid encoding IRG1/ACOD1.
65. The method of any one of embodiments 60 to 64, wherein PGC1α activity is increased by treating the population of mammalian stromal stem cells with at least one of a PGC1α agonist and a nucleic acid encoding PGC1α.
66. The method of any one of embodiments 57 to 65, wherein the method increases the total number of mitochondria in cells of the individual.
67. The method of any one of embodiments 57 to 66, wherein the method increases the total volume of mitochondria in cells of the individual.
68. The method of any one of embodiments 57 to 67, wherein the method increases phosphatidylserine (PS) levels on the cell surface.
69. The method of any one of embodiments 57 to 68, wherein the method increases itaconate levels in the cell.
70. The method of any one of embodiments 57 to 69, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
71. A method of treating an individual, the method comprising administering to the individual a population of mammalian stromal stem cells,
   wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47; and
   wherein at least 30% of the population of mammalian stromal stem cells is positive for SDC2.
72. The method of embodiment 71, wherein the population of mammalian stromal stem cells is at least 30% positive for SDC2.
73. The method of embodiment 71 or embodiment 72, wherein CDC50 activity is reduced by treating the population of mammalian stromal stem cells with at least one of a CDC50 antagonist, a CDC50 inhibitory nucleic acid, and a CDC50 inhibitory peptide.
74. The method of any one of embodiments 71 to 73, wherein IRG1/ACOD1 activity is increased by treating the population of mammalian stromal stem cells with at least one of an IRG1/ACOD1 agonist and a nucleic acid encoding IRG1/ACOD1.
75. The method of any one of embodiments 71 to 74, wherein PGC1α activity is increased by treating the population of mammalian stromal stem cells with at least one of a PGC1α agonist and a nucleic acid encoding PGC1α.
76. The method of any one of embodiments 71 to 74, wherein CD47 activity is reduced by treating the population of mammalian stromal stem cells with at least one of a CD47 antagonist, a CD47 inhibitory nucleic acid, and a CD47 inhibitory peptide.
77. The method of any one of embodiments 71 to 75, wherein the method increases the total number of mitochondria in cells of the individual.
78. The method of any one of embodiments 71 to 77, wherein the method increases the total volume of mitochondria in cells of the individual.
79. The method of any one of embodiments 71 to 78, wherein the method increases phosphatidylserine (PS) levels on the cell surface.
80. The method of any one of embodiments 71 to 79, wherein the method increases itaconate levels in the cell.
81. The method of any one of embodiments 71 to 80, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
82. A method of preparing a population of stromal stem cells, the method comprising:
   a) obtaining a population of mammalian cells;
   b) treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47; and
   c) isolating SDC2+ cells from the treated population of mammalian cells.
83. The method of embodiment 82, wherein the population of mammalian stromal stem cells has reduced CDC50 activity.
84. The method of embodiment 82 or embodiment 83, wherein the population of mammalian stromal stem cells has increased IRG1/ACOD1 activity.
85. The method of any one of embodiments 82 to 84, wherein the population of mammalian stromal stem cells has increased PGC1α activity.
86. The method of any one of embodiments 82 to 85, wherein the population of mammalian stromal stem cells has reduced CD47 activity.
87. The method of any one of embodiments 82 to 86, wherein the population of mammalian stromal stem cells is at least 30% positive for SDC2.
88. The method of any one of embodiments 82 to 87, wherein CDC50 activity is reduced by treating the population of mammalian stromal stem cells with at least one of a CDC50 antagonist, a CDC50 inhibitory nucleic acid, and a CDC50 inhibitory peptide.
89. The method of any one of embodiments 82 to 88, wherein IRG1/ACOD1 activity is increased by treating the population of mammalian stromal stem cells with at least one of an IRG1/ACOD1 agonist and a nucleic acid encoding IRG1/ACOD1.
90. The method of any one of embodiments 82 to 88, wherein PGC1α activity is increased by treating the population of mammalian stromal stem cells with at least one of a PGC1α agonist and a nucleic acid encoding PGC1α.
91. The method of any one of embodiments 82 to 89, wherein CD47 activity is reduced by treating the population of mammalian stromal stem cells with at least one of a CD47 antagonist, a CD47 inhibitory nucleic acid, and a CD47 inhibitory peptide.
92. The method of any one of embodiments 82 to 91, wherein the stromal stem cell is a human cell, a mouse cell, a rat cell, or an equine cell.
93. The method of any one of embodiments 82 to 92, wherein the population of mammalian cells is obtained from a source selected from bone marrow, umbilical cord, umbilical cord blood, adipose tissue, skeletal muscle, endometrium, placenta, Wharton's jelly, and cells derived from pluripotent cells.
94. The method of any one of embodiments 82 to 93, wherein the population of mammalian cells is exposed to a low oxygen environment.
95. The method of any one of embodiments 82 to 94, wherein isolating SDC2+ cells from the treated population of mammalian cells comprises contacting the treated population of mammalian cells to an SDC2 binding agent and isolating cells bound to the SDC2 binding agent.
96. The method of embodiment 95, wherein the SDC2 binding agent comprises an anti-SDC2 antibody or fragment thereof.
97. The method of any one of embodiments 82 to 96, wherein the method increases the total number of mitochondria in the cell.
98. The method of any one of embodiments 82 to 97, wherein the method increases the total volume of mitochondria in the cell.
99. The method of any one of embodiments 82 to 98, wherein the method increases phosphatidylserine (PS) levels on the cell surface.
100. The method of any one of embodiments 82 to 99, wherein the method increases itaconate levels in the cell.
101. The method of any one of embodiments 82 to 100, wherein the method further comprises measuring at least one of IRG1/ACOD1 expression and itaconate release.
102. A method of treating an individual, the method comprising administering to the individual a composition comprising exosomes isolated from a population of mammalian stromal stem cells, wherein the population of mammalian stromal stem cells has reduced expression of CDC50.
103. The method of embodiment 101, wherein the population of mammalian stromal stem cells has increased IRG1/ACOD1 activity.
104. The method of embodiment 101 or embodiment 103, wherein the population of mammalian stromal stem cells has increased PGC1α activity.
105. The method of any one of embodiments 101 to 104, wherein the population of mammalian stromal stem cells has reduced CD47 activity.
106. The method of any one of embodiments 101 to 105, wherein the population of mammalian stromal stem cells is at least 30% positive for SDC2.
107. The method of any one of embodiments 101 to 106, wherein CDC50 activity is reduced by treating the population of mammalian stromal stem cells with at least one of a CDC50 antagonist, a CDC50 inhibitory nucleic acid, and a CDC50 inhibitory peptide.
108. The method of any one of embodiments 103 to 107, wherein IRG1/ACOD1 activity is increased by treating the population of mammalian stromal stem cells with at least one of an IRG1/ACOD1 agonist and a nucleic acid encoding IRG1/ACOD1.
109. The method of any one of embodiments 104 to 108, wherein PGC1α activity is increased by treating the population of mammalian stromal stem cells with at least one of a PGC1α agonist and a nucleic acid encoding PGC1α.
110. The method of any one of embodiments 105 to 109, wherein CD47 activity is reduced by treating the population of mammalian stromal stem cells with at least one of a CD47 antagonist, a CD47 inhibitory nucleic acid, and a CD47 inhibitory peptide.
111. The method of any one of embodiments 101 to 110, wherein the method increases the total number of mitochondria in cells of the individual.
112. The method of any one of embodiments 101 to 111, wherein the method increases the total volume of mitochondria in cells of the individual.
113. The method of any one of embodiments 101 to 112, wherein the method increases phosphatidylserine (PS) levels on the cell surface.
114. The method of any one of embodiments 101 to 113, wherein the method increases itaconate levels in the cell.
*115.* The method of any one of embodiments 101 to 114, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
116. A method of treating an individual, the method comprising administering to the individual a composition comprising exosomes isolated from a population of mammalian stromal stem cells, wherein the population of mammalian stromal stem cells has increased expression of IRG1/ACOD1.
117. The method of embodiment 116, wherein the population of mammalian stromal stem cells has reduced CDC50 activity.
118. The method of embodiment 116 or embodiment 117, wherein the population of mammalian stromal stem cells has increased PGC1α activity.
119. The method of any one of embodiments 116 to 118, wherein the population of mammalian stromal stem cells has reduced CD47 activity.
120. The method of any one of embodiments 116 to 119, wherein the population of mammalian stromal stem cells is at least 30% positive for SDC2.
121. The method of any one of embodiments 116 to 120, wherein PGC1α activity is increased by treating the population of mammalian stromal stem cells with at least one of a PGC1α agonist and a nucleic acid encoding PGC1α.
122. The method of any one of embodiments 117 to 121, wherein CDC50 activity is reduced by treating the population of mammalian stromal stem cells with at least one of a CDC50 antagonist, a CDC50 inhibitory nucleic acid, and a CDC50 inhibitory peptide.
123. The method of any one of embodiments 118 to 122, wherein CD47 activity is reduced by treating the population of mammalian stromal stem cells with at least one of a CD47 antagonist, a CD47 inhibitory nucleic acid, and a CD47 inhibitory peptide.
124. The method of any one of embodiments 116 to 123, wherein the method increases the total number of mitochondria in cells of the individual.
125. The method of any one of embodiments 116 to 124, wherein the method increases the total volume of mitochondria in cells of the individual.
126. The method of any one of embodiments 116 to 125, wherein the method increases phosphatidylserine (PS) levels on the cell surface.
127. The method of any one of embodiments 116 to 126, wherein the method increases itaconate levels in the cell.
128. The method of any one of embodiments 116 to 127, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
129. A method of treating an individual, the method comprising administering to the individual a composition comprising exosomes isolated from a population of mammalian stromal stem cells, wherein the population of mammalian stromal stem cells has increased expression of PGC1α.
130. The method of embodiment 129, wherein the population of mammalian stromal stem cells has reduced CDC50 activity.
131. The method of embodiment 129 or embodiment 130, wherein the population of mammalian stromal stem cells has increased IRG1/ACOD activity.
132. The method of one of embodiments 129 to 131, wherein the population of mammalian stromal stem cells has reduced CD47 activity.
133. The method of any one of embodiments 129 to 132, wherein the population of mammalian stromal stem cells is at least 30% positive for SDC2.
134. The method of any one of embodiments 129 to 133, wherein PGC1α activity is increased by treating the population of mammalian stromal stem cells with at least one of a PGC1α agonist and a nucleic acid encoding PGC1α.
135. The method of any one of embodiments 130 to 134, wherein CDC50 activity is reduced by treating the population of mammalian stromal stem cells with at least one of a CDC50 antagonist, a CDC50 inhibitory nucleic acid, and a CDC50 inhibitory peptide.
136. The method of any one of embodiments 132 to 135, wherein CD47 activity is reduced by treating the population of mammalian stromal stem cells with at least one of a CD47 antagonist, a CD47 inhibitory nucleic acid, and a CD47 inhibitory peptide.
137. The method of any one of embodiments 129 to 136, wherein the method increases the total number of mitochondria in cells of the individual.
138. The method of any one of embodiments 129 to 137, wherein the method increases the total volume of mitochondria in cells of the individual.
139. The method of any one of embodiments 129 to 138, wherein the method increases phosphatidylserine (PS) levels on the cell surface.
140. The method of any one of embodiments 129 to 139, wherein the method increases itaconate levels in the cell.
141. The method of any one of embodiments 129 to 140, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
142. A method of treating an individual, the method comprising administering to the individual a composition comprising exosomes isolated from a population of mammalian stromal stem cells, wherein the population of mammalian stromal stem cells has reduced expression of CD47.
143. The method of embodiment 142, wherein the population of mammalian stromal stem cells has reduced CDC50 activity.
144. The method of embodiment 142 or embodiment 143, wherein the population of mammalian stromal stem cells has increased IRG1/ACOD activity.
145. The method of any one of embodiments 142 to 144, wherein the population of mammalian stromal stem cells has increased PGC1α activity.
146. The method of any one of embodiments 142 to 145, wherein the population of mammalian stromal stem cells is at least 30% positive for SDC2.
147. The method of any one of embodiments 142 to 146, wherein CD47 activity is reduced by treating the population of mammalian stromal stem cells with at least one of a CD47 antagonist, a CD47 inhibitory nucleic acid, and a CD47 inhibitory peptide.
148. The method of any one of embodiments 143 to 147, wherein CDC50 activity is reduced by treating the population of mammalian stromal stem cells with at least one of a CDC50 antagonist, a CDC50 inhibitory nucleic acid, and a CDC50 inhibitory peptide.
149. The method of any one of embodiments 144 to 148, wherein IRG1/ACOD activity is reduced by treating the population of mammalian stromal stem cells with at least one of an IRG1/ACOD agonist and a nucleic acid encoding IRG1/ACOD.
150. The method of any one of embodiments 145 to 149, wherein PGC1α activity is increased by treating the population of mammalian stromal stem cells with at least one of a PGC1α agonist and a nucleic acid encoding PGC1α.
151. The method of any one of embodiments 142 to 150, wherein the method increases the total number of mitochondria in cells of the individual.
152. The method of any one of embodiments 142 to 151, wherein the method increases the total volume of mitochondria in cells of the individual.
153. The method of any one of embodiments 142 to 152, wherein the method increases phosphatidylserine (PS) levels on the cell surface.
154. The method of any one of embodiments 142 to 153, wherein the method increases itaconate levels in the cell.
155. The method of any one of embodiments 142 to 154, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
156. A method of treating an individual, the method comprising administering to the individual a composition comprising exosomes isolated from a population of mammalian stromal stem cells,
   wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47; and
   wherein at least 30% of the population of mammalian stromal stem cells is positive for SDC2.
157. The method of embodiment 156, wherein the population of mammalian stromal stem cells is at least 30% positive for SDC2.
158. The method of embodiment 156 or embodiment 157, wherein CDC50 activity is reduced by treating the population of mammalian stromal stem cells with at least one of a CDC50 antagonist, a CDC50 inhibitory nucleic acid, and a CDC50 inhibitory peptide.
159. The method of any one of embodiments 156 to 158, wherein IRG1/ACOD1 activity is reduced by treating the population of mammalian stromal stem cells with at least one of an IRG1/ACOD1 agonist and a nucleic acid encoding IRG1/ACOD1.
160. The method of any one of embodiments 156 to 159, wherein PGC1α activity is increased by treating the population of mammalian stromal stem cells with at least one of a PGC1α agonist and a nucleic acid encoding PGC1α.
161. The method of any one of embodiments 156 to 160, wherein CD47 activity is reduced by treating the population of mammalian stromal stem cells with at least one of a CD47 antagonist, a CD47 inhibitory nucleic acid, and a CD47 inhibitory peptide.
162. The method of any one of embodiments 156 to 161, wherein the method increases the total number of mitochondria in cells of the individual.
163. The method of any one of embodiments 156 to 162, wherein the method increases the total volume of mitochondria in cells of the individual.
164. The method of any one of embodiments 156 to 163, wherein the method increases phosphatidylserine (PS) levels on the cell surface.
165. The method of any one of embodiments 156 to 164, wherein the method increases itaconate levels in the cell.
166. The method of any one of embodiments 156 to 165, wherein the individual exhibits at least one symptom of a disease selected from a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.
167. A method of preparing a composition comprising exosomes isolated from a population of stromal stem cells, the method comprising:
   a) obtaining a population of mammalian cells;
   b) treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47; and
   c) isolating SDC2+ exosomes from the treated population of mammalian cells.
168. The method of embodiment 167, wherein the population of mammalian stromal stem cells has reduced CDC50 activity.
169. The method of embodiment 167 or embodiment 168, wherein the population of mammalian stromal stem cells has increased IRG1/ACOD1 activity.
170. The method of any one of embodiments 167 to 169, wherein the population of mammalian stromal stem cells has increased PGC1α activity.
171. The method of any one of embodiments 167 to 170, wherein the population of mammalian stromal stem cells has reduced CD47 activity.
172. The method of any one of embodiments 167 to 171, wherein the population of mammalian stromal stem cells is at least 30% positive for SDC2.
173. The method of any one of embodiments 167 to 172, wherein CDC50 activity is reduced by treating the population of mammalian stromal stem cells with at least one of a CDC50 antagonist, a CDC50 inhibitory nucleic acid, and a CDC50 inhibitory peptide.
174. The method of any one of embodiments 167 to 173, wherein IRG1/ACOD1activity is increased by treating the population of mammalian stromal stem cells with at least one of an IRG1/ACOD1agonist and a nucleic acid encoding IRG1/ACOD1.
175. The method of any one of embodiments 167 to 174, wherein PGC1α activity is increased by treating the population of mammalian stromal stem cells with at least one of a PGC1α agonist and a nucleic acid encoding PGC1α.
176. The method of any one of embodiments 167 to 175, wherein CD47 activity is reduced by treating the population of mammalian stromal stem cells with at least one of a CD47 antagonist, a CD47 inhibitory nucleic acid, and a CD47 inhibitory peptide.
177. The method of any one of embodiments 167 to 176, wherein the stromal stem cell is a human cell, a mouse cell, a rat cell, or an equine cell.
178. The method of any one of embodiments 167 to 177, wherein the population of mammalian cells is obtained from a source selected from bone marrow, umbilical cord, umbilical cord blood, adipose tissue, skeletal muscle, endometrium, placenta, Wharton's jelly, and cells derived from pluripotent cells.
179. The method of any one of embodiments 167 to 178, wherein the population of mammalian cells is exposed to a low oxygen environment.
180. The method of any one of embodiments 167 to 179, wherein isolating SDC2+ cells from the treated population of mammalian cells comprises contacting the treated population of mammalian cells to an SDC2 binding agent and isolating cells bound to the SDC2 binding agent.
181. The method of embodiment 180, wherein the SDC2 binding agent comprises an anti-SDC2 antibody or fragment thereof.
182. The method of any one of embodiments 167 to 181, wherein the method increases the total number of mitochondria in the exosomes.
183. The method of any one of embodiments 167 to 182, wherein the method increases the total volume of mitochondria in the exosomes.
184. The method of any one of embodiments 167 to 183, wherein the method increases phosphatidylserine (PS) levels on the exosome surface.
185. The method of any one of embodiments 167 to 184, wherein the method increases itaconate levels in the exosomes.
186. A method of measuring a potency of a batch of stromal stem cells, the method comprising: (a) isolating a population of SDC2+ stromal stem cells from a mixed population of mammalian cells; and (b) measuring at least one of (i) an expression level of IRG1/ACOD1 and (ii) a release level of itaconate.
187. The method of embodiment 186, wherein the expression level of IRG1/ACOD1 is measured by QPCR or quantitative FACS.
188. The method of embodiment 186 or embodiment 187, wherein the release level of itaconate is measured by mass spectrometry.
189. The method of any one of embodiments 186 to 188, wherein the mixed population of mammalian cells is bone marrow cells or umbilical cord cells.
190. The method of any one of embodiments 186 to 189, wherein the mixed population of mammalian cells is human.
191. The method of any one of embodiments 186 to 190, wherein the method further comprises treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47.
192. The method of embodiment 191, wherein the population of mammalian stromal stem cells has reduced CDC50 activity.
193. The method of embodiment 191 or embodiment 192, wherein the population of mammalian stromal stem cells has increased IRG1/ACOD1 activity.
194. The method of any one of embodiments 191 to 193, wherein the population of mammalian stromal stem cells has increased PGC1α activity.
195. The method of any one of embodiments 191 to 194, wherein the population of mammalian stromal stem cells has reduced CD47 activity.
196. The method of any one of embodiments 191 to 195, wherein the population of mammalian stromal stem cells is at least 30% positive for SDC2.
197. The method of any one of embodiments 191 to 196, wherein CDC50 activity is reduced by treating the population of mammalian stromal stem cells with at least one of a CDC50 antagonist, a CDC50 inhibitory nucleic acid, and a CDC50 inhibitory peptide.
198. The method of any one of embodiments 191 to 197, wherein IRG1/ACOD1 activity is increased by treating the population of mammalian stromal stem cells with at least one of an IRG1/ACOD1 agonist and a nucleic acid encoding IRG1/ACOD1.
199. The method of any one of embodiments 191 to 198, wherein PGC1α activity is increased by treating the population of mammalian stromal stem cells with at least one of a PGC1α agonist and a nucleic acid encoding PGC1α.
200. The method of any one of embodiments 191 to 199, wherein CD47 activity is reduced by treating the population of mammalian stromal stem cells with at least one of a CD47 antagonist, a CD47 inhibitory nucleic acid, and a CD47 inhibitory peptide.
201. The method of any one of embodiments 186 to 200, wherein the stromal stem cell is a human cell, a mouse cell, a rat cell, or an equine cell.
202. The method of any one of embodiments 186 to 201, wherein the population of mammalian cells is obtained from a source selected from bone marrow, umbilical cord, umbilical cord blood, adipose tissue, skeletal muscle, endometrium, placenta, Wharton's jelly, and cells derived from pluripotent cells.
203. The method of any one of embodiments 186 to 202, wherein the population of mammalian cells is exposed to a low oxygen environment.
204. The method of any one of embodiments 186 to 203, wherein isolating SDC2+ cells from the treated population of mammalian cells comprises contacting the treated population of mammalian cells to an SDC2 binding agent and isolating cells bound to the SDC2 binding agent.
205. The method of embodiment 204, wherein the SDC2 binding agent comprises an anti-SDC2 antibody or fragment thereof.
206. The method of any one of embodiments 191 to 205, wherein the method increases the total number of mitochondria in the cell.
207. The method of any one of embodiments 191 to 206, wherein the method increases the total volume of mitochondria in the cell.
208. The method of any one of embodiments 191 to 207, wherein the method increases phosphatidylserine (PS) levels on the cell surface.
209. The method of any one of embodiments 191 to 208, wherein the method increases itaconate levels in the cell.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments described herein may be employed. It is intended that the following claims define the scope of the invention and that methods and structures within the scope of these claims and their equivalents be covered thereby.

## Claims

1. A population of SDC2+ stromal stem cells treated with a CDC50A inhibitory nucleic acid for use in inducing immune regulatory monocytes and macrophages.

2. The population of SDC2+ stromal stem cells for use according to claim 1, wherein the immune regulatory monocytes and macrophages are polarized to a M2 phenotype.

3. The population of SDC2+ stromal stem cells for use according to claim 1 or claim 2, wherein the CDC50A inhibitory nucleic acid is a CDC50A shRNA or siRNA .

4. The population of SDC2+ stromal stem cells for use according to any one of claims 1 to 3, wherein the inhibitory nucleic acid is expressed by a virus.

5. The population of SDC2+ stromal stem cells for use according to any one of claims 1 to 4, wherein the population of SDC2+ stromal stem cells has CDC50A expression level reduced by at least 30% compared with an untreated population of SDC2+ stromal stem cells.

6. The population of SDC2+ stromal stem cells for use according to any one of claims 1 to 5, wherein the population of SDC2+ stromal stem cells has CDC50A activity reduced by at least 30% compared with an untreated population of SDC2+ stromal stem cells.

7. The population of SDC2+ stromal stem cells for use according to any one of claims 1 to 6, wherein the population of SDC2+ stromal stem cells has increased cell surface phosphatidyl serine expression compared with an untreated population of SDC2+ stromal stem cells.

8. The population of SDC2+ stromal stem cells for use according to any one of claims 1 to 7, wherein the population of SDC2+ stromal stem cells has increased itaconate in the cells compared with an untreated population of SDC2+ stromal stem cells.

9. The population of SDC2+ stromal stem cells for use according to any one of claims 1 to 8, wherein the SDC2+ stromal stem cells are isolated from umbilical cord or bone marrow.

10. The population of SDC2+ stromal stem cells for use according to any one of claims 1 to 9, wherein the SDC2+ stromal stem cells are human cells.

11. The population of SDC2+ stromal stem cells for use according to any one of claims 1 to 10, wherein the population is formulated in a physiologically acceptable buffer or excipient.

12. A method of preparing a population of stromal stem cells, the method comprising obtaining a population of mammalian cells; treating the population of mammalian cells with a CDC50A inhibitory nucleic acid; and isolating SDC2+ cells from the treated population of mammalian cells.
